# EUROPEAN PATENT SPECIFICATION

(11) **EP 3 649 883 B1**
(45) Date of publication and mention of the grant of the patent: **13.09.2023**
(21) Application number: 19207421.9
(22) Date of filing: 06.11.2019
(51) Int. Cl.: A43D 1/02, B33Y 80/00, A43B 13/22, A61B 5/103, A61B 5/107, B33Y 50/00, A61B 5/11, A61B 5/0205

(54) **A METHOD FOR MANUFACTURING AN OUTSOLE AND A METHOD FOR GENERATING A VISUAL OUSOLE PATTERN**
EIN VERFAHREN ZUR HERSTELLUNG EINER LAUFSOHLE UND EIN VERFAHREN ZUR ERZEUGUNG EINES SICHTBAREN LAUFSOHLENMUSTERS
MÉTHODE DE FABRICATION D'UNE SEMELLE EXTÉRIEURE ET MÉTHODE DE GÉNÉRATION D'UN MOTIF VISUEL DE SEMELLE EXTÉRIEURE

(30) Priority: 09.11.2018 US 201816185954
(43) Date of publication of application: 13.05.2020
(73) Proprietor: adidas AG, 91074 Herzogenaurach (DE)
(72) Inventor: Coupe, Gregory Austin, Portland, OR 97217 (US); Schneider, Andrew Jacob, Portland, OR 97217 (US); Perrault, Jacques, Portland, OR 97217 (US); Berin, Skye B, Portland, OR 97217 (US); Midlam, Gavin Cougar, Portland, OR 97217 (US); Padovani, Matteo Edmond, Portland, OR 97217 (US); Kleiman, Benjamin William, Portland, OR 97217 (US); Rudin, Jake Peter, Portland, OR 97217 (US); Wittchen, Alex Julian, 91074 Herzogenaurach (DE)
(74) Representative: Bardehle Pagenberg Partnerschaft mbB Patentanwälte Rechtsanwälte

(56) References cited:
- US-A- 5 339 252
- US-A1- 2007 039 209
- US-A1- 2015 351 493
- US-A1- 2018 260 645

## Description

### FIELD OF THE INVENTION

The present invention relates to a method of manufacturing an outsole and a method of generating a visual outsole pattern.

### BACKGROUND OF THE INVENTION

Articles of sports apparel such as shoes are usually manufactured as ready-made, mass-produced finished products. They are usually not custom tailored according to measurements, but rather generalized according to anthropometric studies. Thus, athletic shoes are generally made with a single tread pattern, not optimized for a particular individual's needs or to a specific data profile, but with an approximation of the activity and aesthetic considerations (e.g., trail running shoes may have larger lugs and more traction elements than running flats, but neither are informed by objective and customized data).

In professional and ambitious amateur sports, it is known to customize sports apparel up to a certain point. Such customization is often done based on a static analysis, e.g., length and size measurements of the athlete's body. Based on those measurements, customized sports apparel, for example a soccer shoe is then manufactured.

Dynamic analyses are rare. An athlete may be equipped with one or more sensors which are, e.g., attached to one or more of the athlete's limbs while engaged in a typical athletic activity. Video analysis of the athlete engaging in the athletic activity may also occur-a video of the athlete can be recorded and analyzed. For example, a runner may be filmed while running on a treadmill. An orthopedically trained person may then extract information (e.g. supination and pronation) from the filmed video which is then used for manufacturing a customized running shoe having particular cushioning, etc.

However, the mentioned techniques for customizing sports apparel may have disadvantages if used in isolation. These techniques generally require personnel trained in sports medicine and/or orthopedics. And these techniques do not account for traction customization. As such analyses are usually performed during a limited amount of time, they do not show long-term evolution of the athlete's performance characteristic and are prone to day-to-day variations in such performance characteristics-much less variation in the equipment over time, such as a tread wear pattern. Furthermore, as they are generally performed in a laboratory-like environment, such analyses do not provide any context information, i.e. information about the environment in which the athlete typically performs sports activities.

US 2015/ 351493 A1 relates to articles of footwear having sole plates with traction elements thereon. Thereby, the method includes the steps of obtaining/determining one or more input parameters related to the athlete and analyzing the input parameters to determine at least one performance metric of a foot of the user. The performance metric is then used to determine one or more structural characteristics of at least a portion of a sole of an article of footwear for the athlete based on the performance metric, after which the customized sole portion can be manufactured.

### BRIEF SUMMARY OF THE INVENTION

An objective then is producing an article of sports apparel, e.g., a shoe, that is customized for a person, or optimized to a particular data profile, at moderate additional manufacturing costs taking into account long-term variations in performance characteristics and environmental information. Particular advantages can be obtained when providing for individualized traction customization or optimization.

The invention is set out in the appended set of claims.

Deriving pressure data from an individual is also known. However, utilizing light intensity and transforming it into data to accurately determine a wearer's pattern of performance, whether it's banking and cutting in basketball, or sprinting 100 yards (SI Unit: 0.9144 m = 1 yard), is something that has not been perfected.

Indeed, algorithmic solutions disclosed herein may be used such that a data driven input may produce a digital outsole that can be then translated into a physical outsole, with a customized physical traction pattern is based on at least in part a computational traction profile. In this way, sensor and research data on loading profiles for varied running and sports movements (e.g., linear & multidirectional movements) may be applied to produce customized outsoles with improved traction characteristics. For example, data related to direction and magnitude vectors of force or pressure may be applied to alter a digital visual traction pattern in response to the construction of a computational traction profile based on received and analyzed data.

In recognizing that aesthetics may be important to an individual, a novel design of a visual outsole, such as a visual outsole pattern created in a three-dimensional space may be created with attention given to aesthetic and artistic elements. These aesthetic and artistic elements may take the form of traction elements such as projections, protrusions, recesses, apertures, lugs, and other surfaces or features of an outsole. Once a computational traction profile is produced it may be used to alter these aesthetic artistic elements such that they are functionally improved for particular use (e.g., giving the outsole to be produced a more optimized traction characteristic across the outsole).

The digital representation of an outsole (e.g., a visual outsole pattern) may be used to run simulations or open molds and testing prior to production. The visual outsole pattern may be manufactured the one or more manufacturing processes, such as laser cutting, 3-D printing, 3-D molding, etc.

A full digital model may be a three-dimensional model of the apparel (e.g., complete shoe including midsole, insole, upper, last, etc.) including a functional description. The functional description can include information such as which materials are used, where materials are placed, which sensors are used and where sensors are placed, etc.

### BRIEF DESCRIPTION OF THE DRAWINGS

The accompanying figures, which are incorporated herein, form part of the specification and illustrate embodiments of the present invention. Together with the description, the figures further serve to explain the principles of and to enable a person skilled in the relevant arts to make and use the invention.
FIG. 1 shows an outsole data capture system in use by an individual according to an embodiment according to the claimed invention.
FIG. 2 shows a schematic illustration of a traced path performed by an individual on a sensor interface surface of a sensor module according to an embodiment according to the claimed invention.
FIG. 3 shows a schematic illustration of exemplary movements an individual may perform on a sensor interface surface of a sensor module according to an embodiment according to the claimed invention.
FIG. 4 shows a schematic illustration of a sensor module according to an embodiment according to the claimed invention.
FIGS. 5A and 5B show digital output from a sensor module according to an embodiment according to the claimed invention.
FIG. 6 shows variations of computational traction profiles generated by a method according to the claimed invention.
FIGS. 7A and 7B show additional examples of parameters which may be extracted from sensor data generated by a method according to the claimed invention.
FIG. 8 shows examples of traction cutting types generated by a method according to the claimed invention.
FIG. 9 shows a schematic illustration of force data as applied over particular sole elements generated by a method according to the claimed invention.
FIG. 10 shows a system for generating a custom article of apparel according to an embodiment according to the claimed invention.
FIG. 11 shows a flowchart method for generating a custom article of apparel according to an embodiment according to the claimed invention.
FIG. 12 shows a traction pattern generated by a method according to the claimed invention.
FIG. 13 shows a traction pattern generated by a method according to the claimed invention.
FIG. 14 shows a traction pattern generated by a method according to the claimed invention.
FIG. 15 shows a traction pattern generated by a method according to the claimed invention.
FIGS. 16A, 16B, 16C, 16D, 16E, and 16F show traction patterns applied to an outsole, having varying patterns generated by a method according to the claimed invention.
FIGS. 17A and 17B show traction patterns applied to an outsole, having varying patterns generated by a method according to the claimed invention.
FIGS. 18A and 18B show traction patterns applied to an outsole, having varying patterns generated by a method according to the claimed invention.
FIGS. 19A, 19B, 19C, 19D, and 19E show traction patterns applied to an outsole, having varying patterns generated by a method according to the claimed invention.
FIG. 20 shows traction patterns applied to an outsole, having a pattern generated by a method according to the claimed invention.
FIG. 21 shows traction patterns applied to an outsole, having a pattern generated by a method according to the claimed invention.
FIG. 22 shows traction patterns applied to an outsole, having a pattern generated by a method according to the claimed invention.
FIG. 23 shows traction patterns applied to an outsole, having a pattern generated by a method according to the claimed invention.
FIG. 24 shows traction patterns applied to an outsole, having a pattern generated by a method according to the claimed invention.
FIGS. 25A and 25B show traction patterns applied to an outsole, having varying patterns generated by a method according to the claimed invention.
FIG. 26 shows traction patterns applied to an outsole, having a pattern generated by a method according to the claimed invention.
FIG. 27 shows traction patterns applied to an outsole, having a pattern generated by a method according to the claimed invention.
FIG. 28 shows traction patterns applied to an outsole, having a pattern generated by a method according to the claimed invention.
FIG 29 shows an exemplary computer system which can be used for a method according to the claimed invention.
FIG. 30 shows an exemplary sensor module which can be used for a method according to the claimed invention.
FIG. 31 shows an exemplary sensor module which can be used for a method according to the claimed invention.

### DETAILED DESCRIPTION

The present invention will now be described in detail with reference to embodiments thereof as illustrated in the accompanying drawings.

Various aspects of the present invention, or any parts or functions thereof, may be implemented using hardware, software, firmware, non-transitory tangible computer readable or computer usable storage media having instructions stored thereon, or a combination thereof, and may be implemented in one or more computer systems or other processing systems.

As described above, deriving pressure data for an individual is known, for example with instrumented insoles. However, utilizing light intensity and transforming it into data to accurately determine a wearer's pattern of performance, whether it's banking and cutting in basketball, or sprinting 100 yards, is something that has not been perfected. The inventors have provided improved systems and methods related to generating an outsole pattern-and manufacturing said outsole pattern-based on a wearer's profile using a wearer's pressure data and/or step profile.

The phases of the system and process are described herein-initially, an individual's movement pattern is used to generate a profile for the individual. This entails measuring and analyzing a wearer's movement pattern, for example using sensor modules. A frustrated total internal reflection ("FTIR") system is used. A computational traction profile is generated based on data from the FTIR system. Once generated, the computational traction profile is used to generate and/or optimize a visual outsole pattern (e.g., in a 3-D CAD or CAM system).

The visual outsole pattern may be based on at least one characteristic from the individual's profile. For example, parametric modeling may be applied such that the computational traction profile may adjust a shape, size, or number of traction elements to be applied to a visual outsole pattern. Once generated or optimized, the custom visual outsole pattern is translated into a machine language for manufacturing. In this way, the visual outsole pattern is used to manufacture the physical outsole pattern.

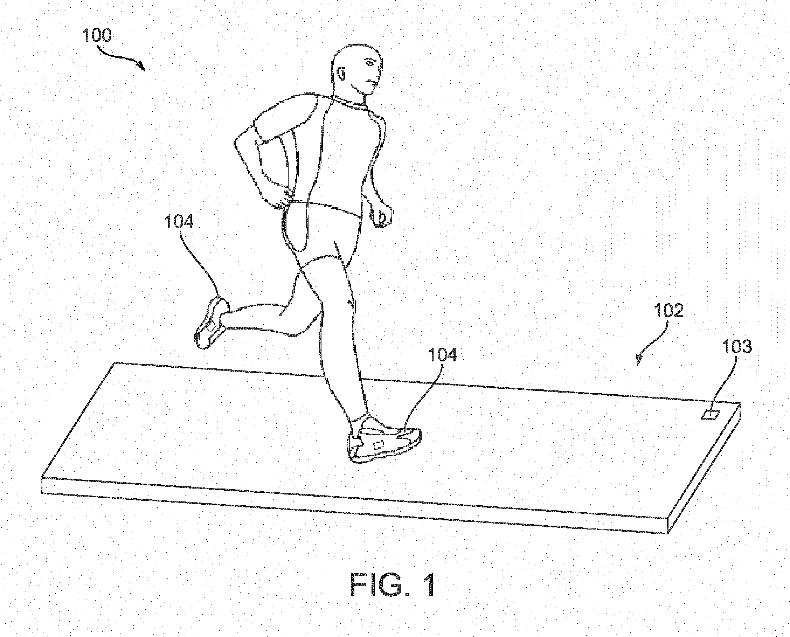

Turning to FIG. 1, individual 100 is shown wearing athletic apparel, e.g. shoe 104. FIG. 1 shows an outsole data capture system in use by an individual 100 according to an embodiment. Individual 100 is shown performing a movement on a sensor module 102. Sensor module 102 is configured as an FTIR system having a floor, or portion thereof. Sensor module 102 may include a communications module 103 that may communicate data obtained by sensor module 102.

During data capture, individual 100 performs one or more movements on the surface of sensor module 102. These movements may include, but are not limited to, running, sprinting, walking, banking, cutting, lunging, squatting, side-stepping, balancing, exercise movements, pronation and supination, foot strike profiles, and hard stop movements. In some embodiments, the movements may correspond to sports specific activities. For example, in the context of basketball, individual 100 may perform game related movements for data capture, such as shooting free throws, jump shots, driving a lane, jump stops, and pivoting.

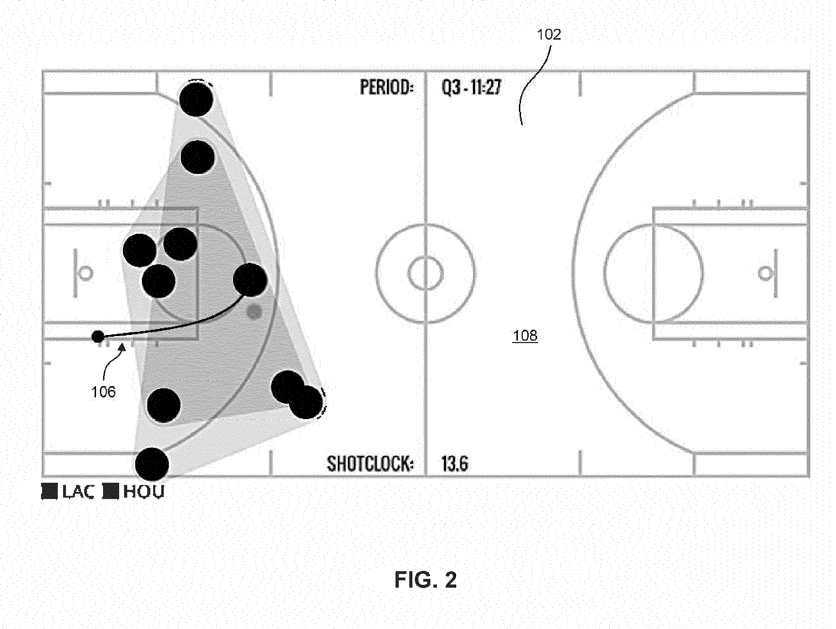

As shown in FIG. 2, a traced path 106 performed by the individual 100 on a sensor interface surface 108 of sensor module 102 is shown, according to an embodiment. The traced path 106 may have portions corresponding to acceleration, deceleration, sidestepping, jumping, for example. Additional data may be provided such as whether the individual 100 is off the ball, on the ball, made a basket, missed a basket, or was fouled, for example. In some embodiments, this data may be derived from game data received from a sensor, or tabulated statistics from a game.

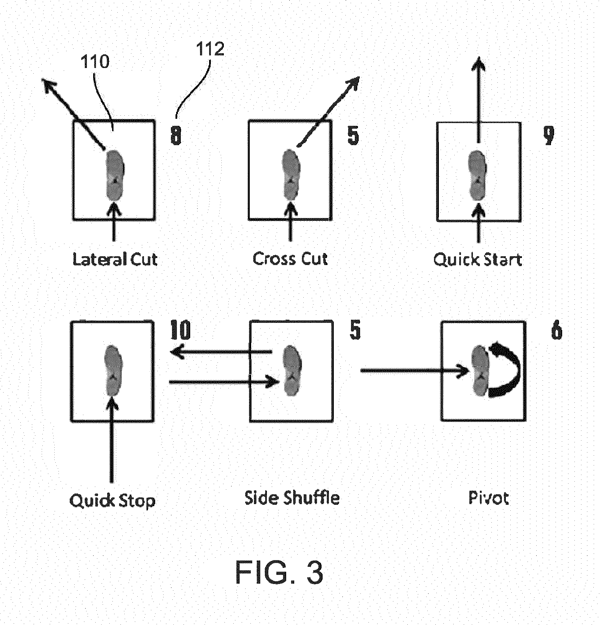

FIG. 3 shows a schematic illustration of exemplary movements an individual may perform on a sensor interface surface of a sensor module according to an embodiment (or in the context of a game). For example, individual 100 may perform a lateral cut, crosscut, quick start, quick stop, side shuffle, pivot. In some embodiments, these and other movements may be associated with instruction icons 110 and may include weighting factors 112 that may be applied in construction of a computational traction profile.

Other movements are contemplated, specific movements such as drives, catching and shooting a ball, picking-and-rolling, off-the-ball screen, pull-up jumper, rebound, etc. In some embodiments, sensor module 102 may be configured as a playing surface for an activity. In some embodiments, sensor module 102 may be embedded in an article of athletic apparel, such as a shoe. Individual 100 may perform movements in a lab setting. In some embodiments, individual 100 may perform movements in an athletic activity setting, for example a sports-specific activity such as a game. Weighting factors 112 may be applied, for instance to denote relative importance of a particular movement to be factored into the computational traction profile of the individual.

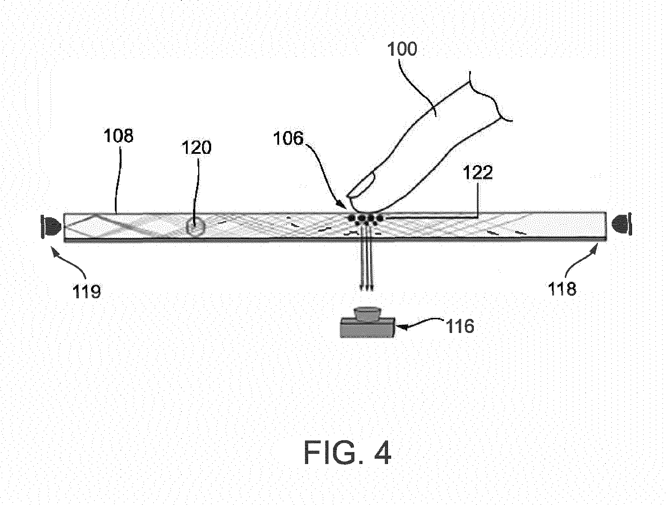

FIG. 4 shows a schematic illustration of a sensor module according to an embodiment. As shown in the figure, sensor module 102 comprises an FTIR system. Shown in the context of an individual 100's finger, sensor module 102 includes a sensor interface surface 108. Sensor interface surface 108 may be a planar surface, such as a floor surface that individual 100 may step, walk, run, jump on, etc. The FTIR system may include an infrared light source 114, which provides light through sensor interface surface 108. The light provided by infrared lighting source 114 travels through sensor interface surface 108 as denoted by the representative light lines. In some embodiments, sensor interface surface 108 may be configured as a panel 120. Panel 120 may be made from a late transmissive surface, such as a plaque of the glass/acrylic surface. As the light travels through panel 120, predictable light behavior is known. Sensor module 102 may further include projection material 118, disposed below panel 120. Further below projection material 118, an infrared camera 116, may be provided.

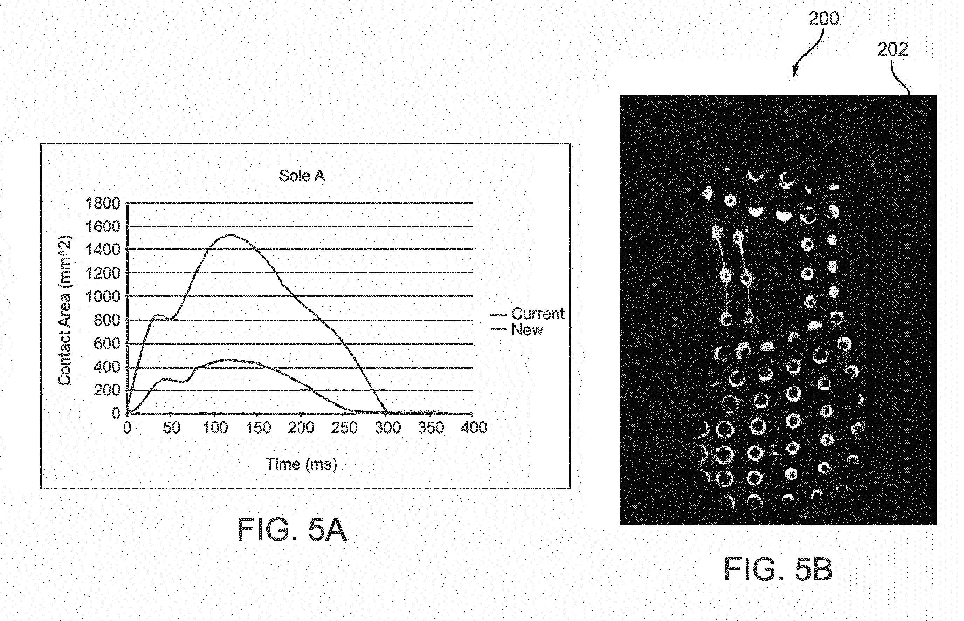

When individual 100, or an article worn by individual 100, such as a shoe, makes contact with sensor interface surface 108, the total internal reflection is frustrated at interface zone 122. Based on the data captured by infrared camera 116 coupled with the principle of critical angle index reflection, the data may be processed to provide a relative profile that may correlate with a gradient pressure map of contact area over a given time when multiple images are captured by infrared camera 116. Examples of such data being captured can be found in FIGS. 5A and 5B.

The frustrated internal reflection occurs when light travels from a material with a higher refractive index in the direction of a lower refractive index at an angle greater than its critical angle. It becomes "frustrated" when a third object comes into contact with the surface and alters the way the waves propagate, and the capture of which may be used to produce a surface pattern. FTIR is able to detect contact area at a very high resolution through image processing. Through software, measured light intensity can be sorted into a gradient of high to low intensity pixels, yielding a measurement of relative pressure. The light intensity data may thus be correlated with traction characteristics of an outsole.

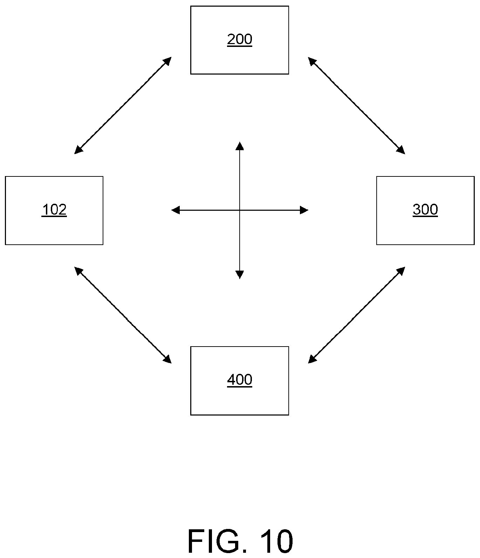

Images captured by camera 116 may be analyzed by a data system 200, further described with reference to FIG. 10, herein. Images of light intensity captured are used to determine values, such as pressure of contact area over time. Light intensity is converted through a calibration process where known loadings and corresponding light intensities are recorded. In this way calibration formula may scale values appropriately for use with the generation of computational traction profiles.

In some embodiments, sensor module 102 may be one or more separate sensors. For example, an instrumented insole (such as those made by Pedar ^{®}) may be provided in addition to a FTIR system. In any case, athlete movement data may be collected, such as plantar pressure distribution data during movements related to that athlete needs. In some embodiments, it can be compared to and used with previous data captured. Types of data which may be analyzed include but are not limited to force, plantar pressure distribution, and direction of movements, such as but not limited to angle and/or magnitude. These movements may correlate with common sports movements.

In some embodiments, on-field/in-game data may be acquired separately from any FTIR analysis. Data acquired by sensor module 102 is used to determine the location of a relative need of traction based on movement requirements and frequency. In some embodiments on-field data may be collected such that various movements are quantified, e.g. by counting which movements are done most often during a game. In this way replication of those movements may be performed within FTIR system not during a game to gather more accurate data. By counting which movements are done most often, key variables may be weighted (e.g., maximum plantar pressure data), and input to produce a computational traction profile that is used in the generation of a visual outsole pattern. Additional examples of techniques that may be used are described in commonly owned U.S. Pat. App. Nos. 13/543,428, filed July 6, 2012, now U.S. Pat. No. 9,317,660, 13/797,361, filed March 12, 2013, now U.S. Pat. No. 9,500,464, 15/016,665, filed February 5, 2016, now U.S. Pat. No. 9,802,080, and15/716,171, filed September 26, 2017 and published as U.S. 2018/0015329.

In addition to the FTIR system other optical contact area measurement systems may be used and disclosed systems and methods. Other mechanical traction tests may also be applied in creating a customized computational traction profile, such as the use of force plates, potentially integrated into an FTIR systems. Other methods, such as slip imaging using high frame rate image subtraction techniques, dynamic center of pressure mapping to better understand loading over time, edge analysis quantifying the number of edges in contact at a given time, and optical shear measurement may also be data input into data system 200 to create the computational traction profile.

Turning to FIGS. 5A and 5B, these figures show digital output from a sensor module according to an embodiment. FIG. 5A shows representative graphic data showing measurement of contact area in square millimeters of a particular outsole pattern compared from a current design to a new design. The x-axis of the graph shown in figure a shows time in milliseconds. Thus, FIG. 5A shows a relatively large gain in contact area over time for a particular step movement when comparing two versions of a particular outsole.

FIG. 5B shows a graphic representation of data captured by infra-red camera 116, showing gradient light indicating their eating degrees of frustrated internal reflection received from the FTIR system. This image can be provided by data system 200, for example displayed on screen 202. The light intensity captured may be coated to show variance in pressure, contact area, etc. For example, an image may show one or more colors corresponding to a relative value such as, e.g., (red denoting high-pressure, green denoting medium pressure, blue denoting low pressure etc.). In some embodiments, these colors may be converted into an intensity measurement, irrespective of their color and may be then used to alter or create the computational traction profile.

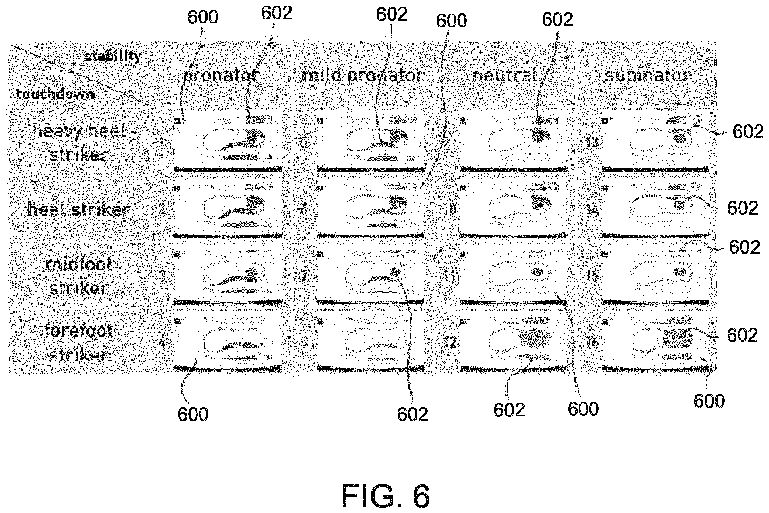

FIG. 6 shows variations of outsole/computational traction profiles according to an embodiment. Information from sensor module(s) 102 may be used to map areas of an individual's foot subject to different pressures or stresses, such as those related to a traction profile. And information from sensor module(s) 102 may be used to generate a computational traction profile. For example, high stress areas may be associated with a heel portion, areas corresponding to the location of the ball of an individual's foot (i.e., at a position corresponding to a location near the anterior end of metatarsals), and a medial most portion of the individual's arch. Mild stress areas may be associated with a medial portion of the individual's arch and areas corresponding to the location of an individual's phalanges. And low stress areas may be associated with a lateral portion of the individual's arch. The size, location, and degree of stress areas for an individual will depend on, among other things, the anatomy of the individual's foot and the individual's gait, and in this regard can be customized even for specific, sports-focused movements. FIG. 6 illustrates sixteen different exemplary computational traction profiles that may be generated based on information from sensor module(s) 102.

In some embodiments, collecting a computational traction profile may include obtaining previously collected and stored data for an individual. In some embodiments, a standard profile for individuals having a certain shoe size, weight, height, arch shape, stability characteristic, and/or touchdown characteristic may be retrieved and then customized based on data obtained through sensor module 102.

In some embodiments, computational traction profiles 600 may be one or more maps generated based on data collected for an individual. In some embodiments, computational traction profiles 600 may be customized profile maps for a group of individuals. For example, computational traction profiles 600 shown in FIG. 6 may be standard biometric profile maps for groups of individuals classified based on four stability characteristics (pronator, mild pronator, neutral, and supinator) and four touchdown characteristics (heavy heel striker, heel striker, midfoot striker, and forefoot striker), which results in sixteen classification groups. As used herein a "stability characteristic" refers to how an individual's foot rolls when it contacts the ground and a "touchdown characteristic" refers to how an individual's foot strikes the ground.

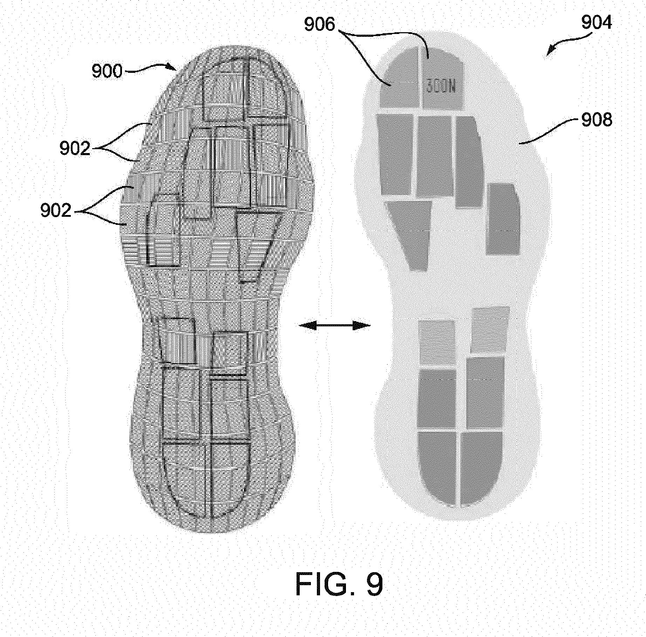

Computational traction profile 600 may include various stress areas 602 associated with a particular individual 100. Computational traction profiles 600 may include various stress areas 602 associated with different groups of individuals, based on information from sensor module(s) 102. For example, as shown in FIG. 6, certain combinations of stress areas 602 may be associated with a heavy heel striker/pronator, a certain combination of stress areas 602 may be associated with a heavy heel striker/mild pronator, a certain combination of stress areas 602 may be associated with a heavy heel striker/neutral foot roll, and so on. Stress areas 602 may be high stress areas, mild stress areas, or low stress areas typically associated groups of individual. And each of the sixteen classification groups may be associated with a particular combination of stress areas 602, which may correlate with a traction characteristic desired. In some embodiments, data collected from sensor module(s) 102 for a particular individual may be utilized to assign the individual a standard biometric data profile map or computational traction profile best suited to that individual. These computational traction profiles may further be customized with data from sensor module 102 as described herein. Indeed, these profiles along with another information collected about an individual (e.g., athletic goals, type of activity, traction data from sensor module 102, etc.), may be used to create a more detailed profile (e.g., shown in FIG. 9).

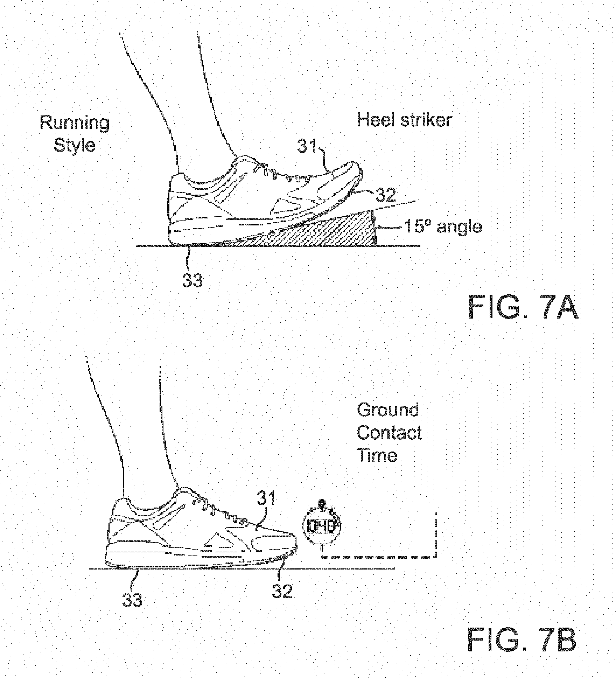

Turning to FIGS. 7A and 7B illustrate additional parameters which may be extracted from sensor data to manufacture the customized apparel, e.g., a shoe. The sensor data may be obtained by at least one sensor integrated into sports apparel while it is worn during a sports activity by the individual. Also, the sports activity may be different and may for example be running, hiking, soccer, rugby, football, basketball, volleyball, tennis, track, field training, cycling, swimming and the like. Additional examples of parameters which may be input into the computational traction profile are described in commonly owned U.S. Pat. App. No. 15/416,185, filed January 26, 2017, and German Patent App. No. 102016201151.0, filed January 27, 2016.

As illustrated in FIG. 7A, one parameter which may be extracted from sensor data is the running style of the person. In this example "running style" refers to the angle of the bottom side of the outsole 32 of the shoe 31 relative to the ground 33 when the foot touches the ground. In the example of Fig. 7A the angle is 15°. Therefore, the person is considered to be a "heel striker". In this case, the customized sports shoe manufactured according to a digital model based on the sensor data may comprise more cushioning in the area of the heel and/or the profile or shape of the outsole, for example in in the area of the heel. This data may also feed into the custom traction elements that are input into the computational traction profile in order to create the visual outsole pattern. "Running style" may also refer to whether the person overpronates, supinates, or is a neutral runner. In general, the running style may be detected by a combination of an accelerometer and a gyroscope (plus magnetometer in certain applications) or by a combination of accelerometer and magnetometer or by an accelerometer alone.

In order to be able to measure the angle of the outsole at the point of time when the heel hits the ground 33, the running shoe 31 may be equipped with an accelerometer and a gyroscope. Data from the accelerometer allows to determine the impact of the heel on the ground, whereas data from the gyroscope allows to determine the orientation of the shoe during such impact.

FIG. 7B illustrates a further parameter which may be extracted from sensor data, namely the ground contact time which corresponds to the duration of time the outsole 32 of the shoe 31 contacts the ground 33 during a gait cycle. To measure ground contact time, an accelerometer may be used to detect time periods in which the shoe 31 is at rest, such as during ground contact. Alternatively, or - additionally, data from a pressure sensor in the outsole, midsole or insole may indicate when the foot is resting on the ground.

Typically, ground contact time can give the runner a feedback about his running style. Based on the ground contact time it also possible to derive, possibly based on a calibration, the speed, distance, and/or pace of the person. These parameters can be used to let the person know when the shoe is worn out or to provide the person with performance data, like speed, distance, pace, etc. Furthermore, during times of no contact with the ground, a processor in the shoe may be put into sleep mode, and battery power can be conserved.

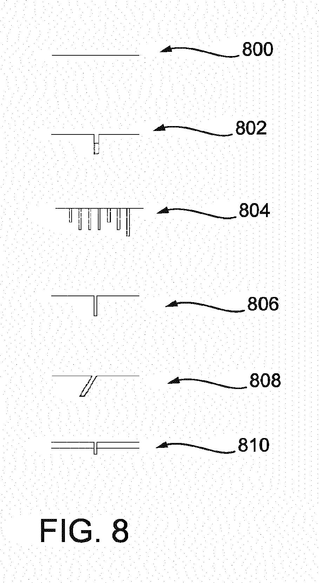

These parameters may inform the type of friction element to be used in an outsole. These friction elements may be applied during the generation of the computational traction profile, which in turn is used to generate a visual outsole pattern, and finally a physical outsole. Turning to FIG. 8, exemplary traction patterns are shown that may be cut or otherwise formed from or onto a surface, for example as a laser cut process.

Starting at the top of FIG. 8, one mold traction pattern 800 is shown. Traction pattern 800 may be a flat pattern and may repeat along the surface of an outsole. Traction pattern 802 is shown below, showing a laser cut traction element. The laser cut traction element may be cut from a flat outsole, as determined by the visual outsole pattern generated from the custom computational traction profile. Traction pattern 802 may be cut, or recut, for example if the traction pattern becomes worn or ineffective. In some embodiments, traction pattern 804 may be used, which shows a modular cut pattern. Modular cut pattern 804 may be particularly useful in creating outsole based on the visual outsole pattern generated by the custom computational traction profile. As shown in the figure, traction pattern 804 may have varying degrees of laser cut elements in the outsole. Traction pattern 806, traction pattern 808, and traction pattern 810 each show additional variations on laser cut traction patterns. Traction pattern 810 shows the cut through a midsole. Variables which can affect traction of outsole designs include the number of edges, compliance of rigs, height, width, and direction of edges. Traction response is generally dependent on directionality and loadings. Depths of cuts are based on pressure data/vector orientation/vector depth. Controlling these variables allows for control and optimization of traction properties for different and unique movements. One or more of each of the traction pattern shown in FIG. 8 may be used, for example in a laser cut outsole. Other manufacturing techniques are contemplated, for example molding, over molding, 3-D printing, patching, two or three dimensional thermo-molding, injection molding, rubber molding, etc.

FIG. 9 shows a representation of an outsole matrix 900, along with a computational pressure matrix 904. Using such matrices, it is possible to obtain a series of pressure maps (similar to so called "heat maps") that encode the dynamics of how the foot behaves while moving, e.g., the distribution of pressure over time, as received from sensor module 102. The maps may include elements 902 that correspond to a particular traction element. As shown, different elements in the pressure matrix 904 may include relatively higher-pressure regions 906 and regions with relatively low pressure 908. Each of these matrices may correspond to a computational traction profile, be used to create or alter a computational traction profile, or serve in and of itself as a computational traction profile.

Taken together, computational pressure matrix 904 includes a plurality of different zones located, sized, and shaped to provide desired characteristics. The zones may be correlated to a particular traction pattern and may be made from one or more elements. Once a computational traction profile is obtained and built using data from sensor module 102, a visual outsole pattern is generated based on the profile. The visual outsole pattern may be generated using computer modeling program such, as but not limited to Grasshopper 3D, Rhinoceros 3D, Modo 3D CAD software or the like.

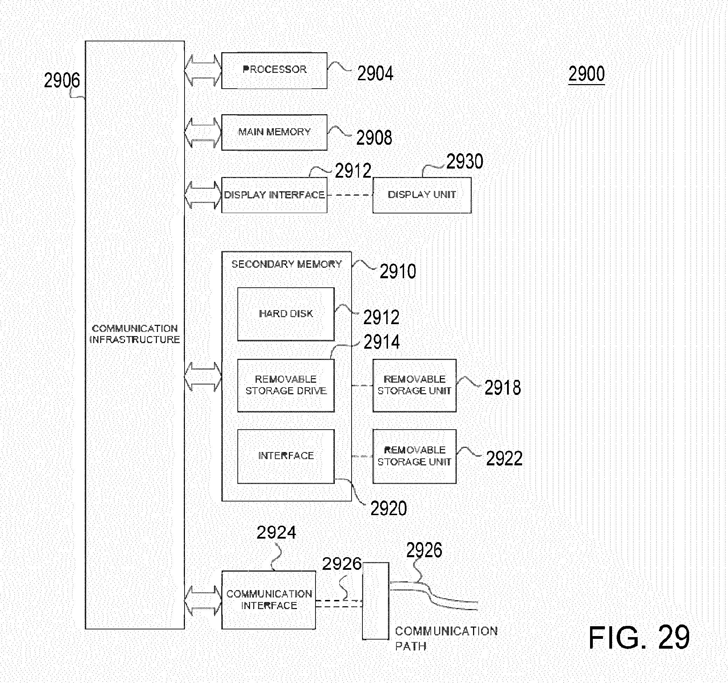

FIG. 10 shows a general system for creating custom outsoles. The system includes a sensor module 102 and data system 200. Sensor module 102 and data system 200 may be configured to communicate with one another, for example through a transceiver. Each of the elements shown in figure 10 may communicate with one another, in a single or bidirectional manner. In some embodiments, not all components need be in communication with one another. The system further includes data translation system 300, and manufacturing system 400. In general, transferring data between the various systems can be performed via Bluetooth, Bluetooth Low Energy (BTLE), Wi-Fi, NFC, a cellular network or the like. Also, it is possible to transmit the data via a wired connection such as a USB or a serial connection for example. Each of the sensor module 102, data system 200, data translation system 300, manufacturing system 400 may comprise one or more processors, computers, servers, etc., for example as described in FIG. 29.

Data system 200 may receive data from sensor module 102, for example FTIR data. This data may be captured and stored for analysis by data system 200. Data system 200 may utilize computational pressure matrices or pressure map, and data from sensor module 102 in order to create a customized computational traction profile. In this way, measuring and analyzing an individual's movement pattern can be applied to the creation of the visual outsole pattern, further optimizing an outsole produced for a particular traction profile.

Data system 200 may evaluate data from sensor module 102. For example, the sensor module 102 data may be preprocessed to remove noise, identify particularly relevant sections of the sensor data corresponding to sports activities, etc. To this end, techniques of preprocessing, digital filtering, feature extraction, statistical processing, machine learning, etc. may be used. In the case of using additional data outside of FTIR, data system 200 may also derive information from the sensor module 102 data such as usage (e.g. lifetime distance) of the previous product (e.g. shoe), average speed on a run, difference in altitude, average pace of the shoe, maximum speed, temperature in the product (e.g. shoe) or on the skin of the person, pressure points within the product (e.g. shoe), etc. System 200 may include components such as a processor, microcontroller, ASIC, DSP, etc., to carry out such processing and analysis, or the analysis may be performed on a different device, e.g. a server, desktop computer, cloud server, etc. In the latter case, the sensor data may have been transmitted to the different device as described above.

Data translation system 300 may receive processed data from data system 200, including a computational traction profile. Then, data translation system 300 may generate or optimize a visual outsole pattern. As described above, the visual outsole pattern may be generated using computer modeling program such, as but not limited to Grasshopper 3D and/or Rhinoceros 3D CAD software. In some embodiments, the software is used to translate and convert the data to geometries for outsole patterns. In some embodiments images may be converted to a color image, for example an RGB image. In this way colors may correspond to estimated force, pressure, service area values, etc. data may be translated via the images brightness, for example converting the RGB values into a single value. Data translation system 300 may then use these values to generate, or optimize, local elements of a visual outsole pattern. In this way, particular elements may be shaped, sized, and placed according to local optima for traction purposes.

Machine instructions may be provided by data translation system 300, or manufacturing system 400. In this way, a physical outsole may be produced based on generated visual outsole pattern provided by data translation system 300. Manufacturing system 400 may include machine instructions, and/or manufacturing components required for producing a physical outsole based on the optimized computational traction profile. Several different manufacturing methods are contemplated and may be used alone or together to create a physical outsole. In some embodiments, outsole pattern may be molded onto a block outsole blank. In some embodiments, laser-cutting or etching may be used such that slip-like patterns may be cut into a block outsole blank. In some embodiments, thermo-molding or similar processes may be used to create the physical outsole. In the case of thermo-molding, the outsole may be molded on a relatively flat surface, or three-dimensional form such as a last. In some embodiments, an outsole may be 3-D printed. Additional examples of manufacturing techniques that may be used are described in commonly owned U.S. Pat. App. Nos. 15/156,062, filed May 16, 2016 and published as U.S. 2017/0325545, 15/156,104, filed May 16, 2016 and published as U.S. 2017/0325546, 15/470,570, filed March 27, 2017 and published as U.S. 2018/0271213, 15/898,000, filed February 15, 2018 and published as U.S. 2018/0271211, and 15/588,433, filed May 5, 2018 and published as U.S. 2018/0317606.

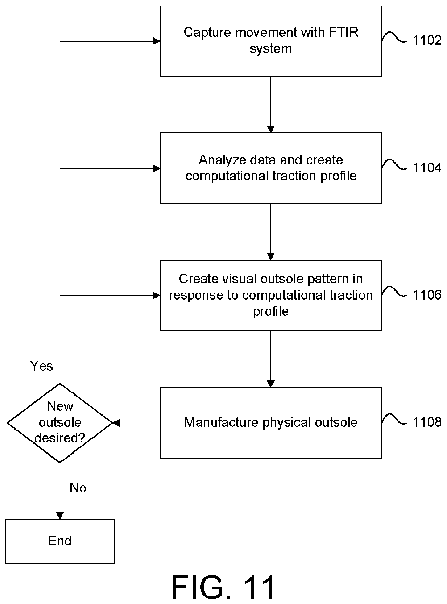

FIG. 11 shows a general method 1100 for creating custom outsoles, e.g., with the system including one or more elements of FIG. 10. At step 1102, movement is captured within FTIR system. At step 1104, the data is analyzed and a computational traction profile is created. At step 1106, a visual outsole pattern is created in response to the computational traction profile. At step 1108 a physical outsole is manufactured.

At this time a decision may be made as to whether a new outsole is desired and if so revert back to one or more of the previous method steps 1102, 1104, 1106, or 1108. So, if updates or refinements to the outsole are required new updates and changes can be made in a new outsole produced. In some embodiments, a finished product such as a shoe may be produced having a custom outsole based on the computational traction profile as well as an instrumented insole, for example pressure sensitive insole that may measure or augment pressure mapping data during use. In some embodiments, this data may be fed into the method 1100 at one or more points along the method steps.

In some embodiments, the base type of outsole may be selected by an individual for production. For example, the standard traction profile may be selected based on factors including but not limited to type of sport/activity, type of foot profile, body type, weather, environment, type of surface, type of outsole material, type of footwear, type of manufacturing, gait characteristics, degree of waterproofness desired, mechanical load requirements, thickness, size, weight, etc. Once the standard traction profile selection has been made to produce a customized outsole, the computational traction profile may be used to customize the visual outsole pattern. In some embodiments, the shape, design, dimensions of the traction profile, etc., and are manipulated based on the received data within the computational traction profile.

In some embodiments, the outsole is formed from a material that may be cut, e.g., laser cut, such as rubber, thermoplastic polyurethane (TPU), polymeric materials, and the like. In this way, an outsole blank may be produced having general contours of footwear, without any traction pattern cut into them. Once the computational traction profile is produced in step 1104 and a visual outsole pattern is created at step 1106, the pattern may be laser cut into an outsole blank, thus producing a finished outsole. In some embodiments, additional testing (e.g., FTIR testing, biomechanical testing, or mechanical testing (e.g., wear life of the produced outsole) may be conducted. In some embodiments, results can be compared to prior studies or based on new criteria-for example the use of inshoe measuring devices such as instrumented insoles in order to evaluate the effectiveness of the finished outsole having the custom traction pattern.

In some embodiments, based on testing with a created physical outsole, treatments may be made to traction pattern by feeding data into method steps 1102, 1104, 1106, or 1108. Once improvements are made, the same physical outsole may be recut, e.g. via a laser cutting process. In this way, the custom computational traction profile may take advantage of and incorporate new information from data collected, may change relative weighing of variable importance, and may investigate additional, new variables for testing and incorporation into the computational traction profile. In this way, individualized outsoles may be produced and improved upon. Indeed, the traction patterns produced by the systems and methods disclosed herein may achieve higher degrees of traction for particular movements than otherwise possible with traditional outsole design.

Advantageously, through this operation no new mold need be created. Testing, optimization, and production of the finished outsole, or even finished article of footwear, may be completed for example at a point-of-sale location such as a retail store. This simplifies the supply chain and decreases shipping costs, e.g. overseas shipping. Additionally, embodiments that use laser cutting to produce the outsoles, environmental gains are possible in that as the outsole begins to wear down an individual may take the outsole to the manufacturing location (or otherwise ship it to the manufacturing location) and this sole traction pattern may be recut, without using a new, fresh outsole blank. Other methods of cutting that may provide similar advantages include water cutting, knife cutting, and needle cutting.

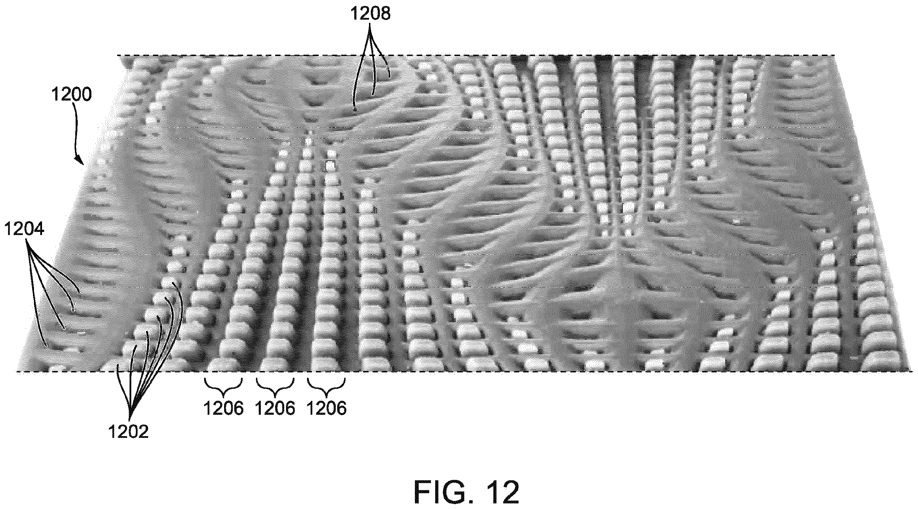

In some embodiments, the pattern of the tread may be adjusted to account for variables such as areas of high wear, high traction requirements, directional loading requirements (e.g., athlete needs), etc. In response to these variables changing, features of a particular visual outsole pattern may be adjusted. For example, traction elements may be raised, lowered, changed in size, and changed in shape. Moreover, the thickness of a given region of an outsole may be adjusted, for example to reduce abrasion. These and other varying features are described further with reference to FIGS. 12-28.

With reference to FIGS. 10 and 11, in some embodiments, an individual may select an initial design of a product to be manufactured. Different parameters can influence the design of the product, such as the cultural context of the person for which the product is customized and manufactured, current trends, user preferences, location of the user and social data. In some embodiments, an individual may select a type of shoe to be produced. In some embodiments, the type of outsole may define an available shoe, or vice versa.

In some embodiments, selection of the outsole is based at least in part on received sensor data obtained by at least one sensor module 102. Individual 100 may select an existing sole material (such as EVA, eTPU, ePEBA, TPU). As described above, various parameters may be used to suggest which type of outsole blank or standard outsole to begin with, prior to customizing a physical outsole using the custom computational traction profile to create the visual outsole pattern. For example, if the person is outdoors most of the time, an outdoor outsole may be pre-selected. If the person is indoors most of the time, an indoor outsole may be pre-selected. If the shoe is exposed to a lot of water a waterproof/water resistant outsole may be pre-selected. If the person is a forest runner, a thin outsole may be pre-selected as forest ground is flexible. If the person is a street runner, a thick outsole may be pre-selected as resilience is required. If the person is a heel striker, the heel of the outsole may be stronger. If the exact size of the foot of the person is known the outsole can be manufactured according to this size and is not limited to traditional, discrete shoe sizes.

In some embodiments, bending of the outsole, e.g., its general stiffness may be selected based on one or more parameters. For example, whether the user is a forefoot or heel striker and whether they typically perform indoor or outdoor sports may influence the stiffness of the outsole. The bending of the shoe may be adjusted by an appropriate placing of patches-for example as described in coowned German Pat. App. DE 10 2015 224 885. In order to complete an article of footwear having a custom physical outsole, other components may be selected either automatically or through user input, such as an insole, upper, etc., each of which may be further customized to produce a custom article footwear for individual 100. As described above, additional details of component selection for a finished footwear product they be found in commonly owned U.S. Pat. App. No. 15/416,185, filed January 26, 2017, and German Patent App. No. 102016201151.0, filed January 27, 2016.

In some embodiments, a biometric data profile may be collected using a physiological and personal characteristic collection and analysis system. In some embodiments, the biometric data profile may be collected using the data collection and analysis system described in U.S. Pat. App. No. 14/579,226, filed on December 22, 2014 and published as US 2016/0180440.

In some embodiments, the custom shoes with customized outsoles may be provided by a locker outfitter service system. For example, if the person forgot his/her sport clothes but has an urgent need for them, the locker outfitter service may deliver the needed sports shoes, to the desired location (e.g. a gym) within a certain period of time (e.g. 8 hours). To this end, the sports shoes may be produced (e.g. by using fully automated production techniques including 3D printing, placement of components by robots, knitting machines, etc.) based on a digital model that includes a custom computational traction profile in, which may be stored in a database, server, computer, or cloud for example. The shoes may for example be produced in a shop or retail store near the person's current location to minimize delivery delay. A customized sports shoes according to the invention may be obtained-the product indeed may be a customized sports shoe. However, the present invention is not limited to sports shoes. Other footwear is contemplated.

One or more of the steps in method 1100, or system shown in FIG. 10, may be performed on a mobile device (e.g. a mobile phone, smartwatch, tablet computer, etc. of the person), a computer of the person (such as a desktop computer or laptop), a computer in a shop/retail store. The selection of the desired product could be saved locally on the particular device and/or on a server and/or in a cloud. "Cloud" in the context of the present invention is understood as the storing of data in a remote computing center, but also the execution of programs which are not installed on a local device (e.g. smartphone, smartwatch, tablet computer, notebook, desktop computer, etc.) or server, but in the cloud.

Once the visual outsole pattern is created, e.g., as a digital model in a CAD or CAM system, the outsole may be created and integrated into a custom article of footwear. The model may comprise information about which materials are used, their shape and dimensions, the kind and number of traction elements, and their placement on the outsole. In some embodiments, updating an existing digital model may be performed prior to or after manufacturing the outsole based on the computational traction profile.

In some embodiments, the individual may search in a database for existing shoe models or may get recommendations of existing shoe from the system. The parameters of the selected shoe (e.g. shape of the shoe, materials used, patch placement, etc.) may then be updated based on the sensor module 102 data obtained. In some embodiments, more than one custom outsoles may be manufactured, having a different traction pattern. However, each traction pattern may be customized using the computational traction profile that alters the visual outsole pattern.

The details for the article such as the shoe may also be customized according to the user's input. For example, the person may be asked which sensor(s) to include. Design comment such as colors, logos, etc. may also be customized. In some embodiments, functional properties may be customized (e.g. waterproofness, cushioning, etc.). In step 1108, a customized product (e.g. a shoe) is produced based on the digital model. This allows the shoe to be manufactured by partially or fully automated production techniques including 3D printing, placement of components by robots, knitting machines, weaving, braiding, etc. Such techniques are able to produce customized sports shoes at moderate costs and high throughput. Furthermore, it is possible to manufacture such customized sports shoes based on digital models not only in a factory, but also e.g. in a store. Additionally, manual methods of constructing such as an outsole or finished product such as a shoe are contemplated.

The customized sports shoe obtained in step 1108 may be equipped with at least one sensor module 102, in this case a sensor module such as an instrumented insole, accelerometer, or the like. Thus, this shoe may be then used in sports activities to obtain additional sensor data for a next generation of the product (e.g. a sports shoe) with further improved and customized characteristics. By this iterative process, the person may obtain a better product in each generation of products. Furthermore, the products may adapt to variations of the person's characteristic over time.

In some embodiments, an individual may order a sports shoe on a website of a sports equipment distributor. That individual may already have login data from a previous purchase and enters this login data, e.g. user name and password into a user interface such as a graphical user interface. The individual may be identified by his/her username or other appropriate identification information, such that client data may be loaded into one or more of a sensor module 102, data system 200, data translation system 300, and manufacturing system 400. The client data may be loaded from a database maintained by the sports equipment distributor or from a server which may be located in a cloud-based storage solution. This database may store all of the user data as described, including for example previous shoes constructed, individual computational traction profiles associated with the individual, a visual outsole patterns of previous outsoles created, and the like. This and other user data may form the basis for building the new shoe model, and physical shoe.

In some embodiments, gait cycle analysis may be performed in the shoe itself. In this case, a CPU may be integrated into the shoe which is capable of performing more advanced calculations as need by a gait cycle analysis. It is alternatively possible to store the sensor data in a memory in the shoe, transmit (e.g. via Bluetooth, BTLE, Wi-Fi, NFC, a cellular network transceiver or other transmission protocols) the sensor data to a different device (like a smartphone, table computer, desktop computer, server computer, cloud computer, etc.) and have the gait cycle analysis done there.

In some embodiments, a 3D view of the shoe model, or even simply be visual outsole pattern may be presented to the person on a display screen, for example in a window of a web browser or another suitable program (e.g., in an app running on a smartphone, smartwatch, tablet computer, digital media player, laptop computer, desktop computer or the like).

The customized sports shoe may be equipped with pronation support, supination support, or may be a neutral running shoe. Furthermore, the customized shoe can also be built with specific insole, midsole and/or outsole material, specific cushioning material in specific areas or a specific profile on the outsole, all based on the data received by sensor module 102 and adapted to the computational traction profile generated. For example, the insole may be made from a soft foam material, a stiff foam material, or a combination of both, eTPU/ePEBA material, EVA material, or any combination of several materials. Likewise, the midsole may be made from a soft foam material, a stiff foam material, or a combination of both, eTPU/ePEBA material, EVA material, or any combination of several materials. The outsole may be made from rubber having certain properties, e.g. sticky, non-marking, non-sticky, etc.). Different combinations of material and/or material properties may be used for the outsole.

FIGS. 12-15 show exemplary planar traction patterns 1200, 1300, 1400, and 1500 according to some embodiments, each of which may be applied to an outsole, and each of which may be modified based on computational traction profile and tailored to specific movements.

Turning to FIG. 12, exemplary planar traction pattern 1200 is shown. Planar traction pattern 1200 may include a surface extending along a given direction. In some embodiments, planar traction pattern 1200 may include a projection 1202 extending from a reference plane on the surface. In some embodiments projections 1202 may be organized into one or more arrays 1206. Arrays 1206 may be linear or nonlinear in nature and may take the form of complex shapes and curves/splines across planar traction pattern 1200. In some embodiments, projections 1202 may be of uniform shape and/or size or may vary along planar traction pattern 1200.

In some embodiments, planar traction pattern 1200 may include recesses 1204. Recesses 1204 similarly may be organized into arrays and may be disposed protruding inward into planar traction pattern 1200. In some embodiments, recesses 1204 may be of uniform shape and/or size or may vary along planar traction pattern 1200. Recesses 1204 may vary in depth, width, and length for example. In some embodiments, various clusters of recesses may be formed in different arrangements. Recesses 1204 may be formed as slits, concave features, apertures, or through holes. In some embodiments, projections 1202 may be disposed within recesses 1204 and may provide a tooth like visual impression. In some embodiments, recesses 1204 or projections 1202 may be cut, such as laser cut, embossed, engraved, or the like.

In some embodiments, planar traction pattern 1200 may include a fin element 1208 extending from a reference plane on the surface. In some embodiments, fin elements may be of uniform shape and/or size or may vary along planar traction pattern 1200. As with projections 1202 and recesses 1204, fin elements 1208 may be formed in arrays, may be linear or nonlinear and structure, and may take the form of complex shapes and curves/splines across the planar traction pattern 1200. In some embodiments, the arrangement of these features may be a repeating or nonrepeating pattern along planar traction pattern 1200. In some embodiments, the pattern may be symmetric or asymmetric about a particular axis.

Certain elements of planar traction pattern 1200 may be fixed in a particular geometry or shape in some embodiments and are not altered by a generated computational traction profile in some embodiments. Alternatively, some embodiments, some or all of the elements of planar traction pattern 1200 may be altered by computational traction profile, prior to finalizing a visual outsole pattern and manufacturing the physical outsole. As used herein, "planar" may generally refer to an extended surface, and may not necessarily reflect only a flat plane, that is, the planar traction pattern 1200 may be curved, rent, angle, warped, etc. in three-dimensional space. In some embodiments, this shaping of planar traction pattern 1200 may depend in part or in whole on the computational traction profile. For example, projections may be raised in particular zones and recesses may be lowered in others, for example.

Some or all of the elements of planar traction pattern 1200 may be grouped together in different arrangements, for example a larger group of recesses 1204 may be disposed in one region as opposed to another. In some embodiments, some or all of the elements of planar traction pattern 1200 may be interconnected to form a continuous pattern. In some embodiments, some or all of the elements of planar traction pattern 1200 may be intermittent. Some embodiments one or more continuous patterns may be formed from some or all of the elements planar traction pattern 1200. One or more of the elements of planar traction pattern 1200 may be formed from various polygon shapes. In some embodiments, the frequency of the elements disposed on planar traction pattern 1200 may vary along the surface of planar traction pattern 1200.

Some or all of the features in each traction profile shown in the figures, including those shown in whole or partial outsole embodiments, may be applied in each of the other figures without limitation. For example, features, elements, and properties discussed with reference to planar traction pattern 1200 may be used in planar traction pattern 1300, or in outsole 1600, for example.

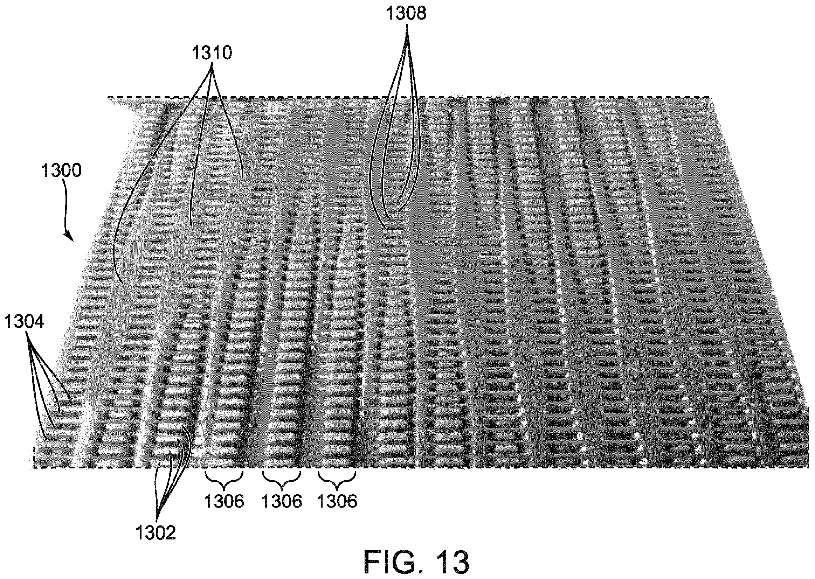

Turning to FIG. 13, exemplary planar traction pattern 1300 is shown. Planar traction pattern 1300 may include a surface extending generally in a given direction. In some embodiments, planar traction pattern 1300 may include a projection 1302 extending from a reference plane on the surface. In some embodiments projections 1302 may be organized into one or more arrays 1306. Arrays 1306 may be linear or nonlinear in nature and may take the form of complex shapes and curves/splines across planar traction pattern 1300. In some embodiments, projections 1302 may be of uniform shape and/or size or may vary along planar traction pattern 1302. Recesses 1304 may also be included. Planar traction pattern 1300 may include one or more extended surfaces 1310. Extended surface 1310 may be raised or lowered relative to the general plane of the traction pattern. In some embodiments, the thickness of the planar traction pattern may be altered in response to the computational traction profile. One or more of the extended surfaces may gradually rise along a recess.

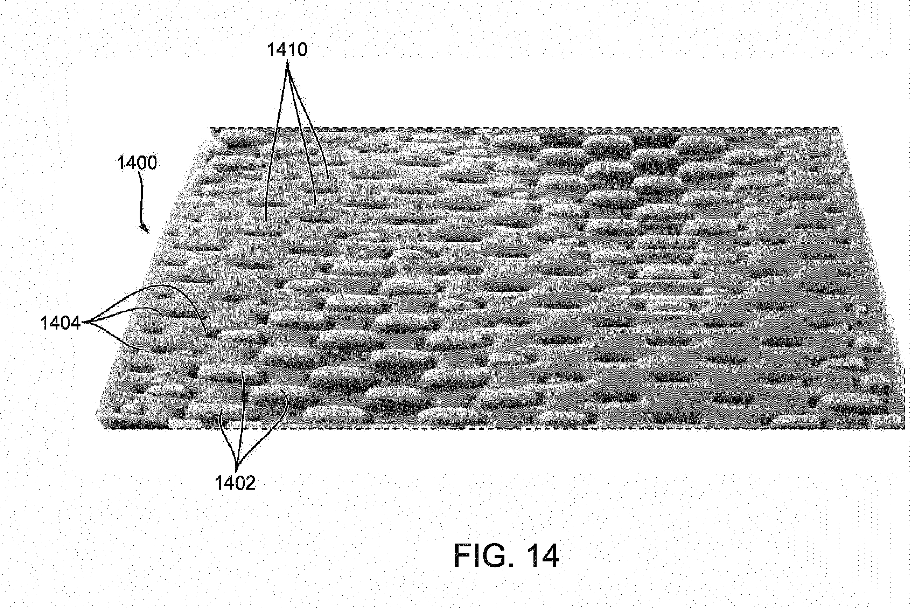

Turning to FIG. 14, exemplary planar traction pattern 1400 is shown. Planar traction pattern 1300 may include a surface extending generally in a given direction. In some embodiments, planar traction pattern 1400 may include a projection 1402 extending from a reference plane on the surface. In some embodiments projections 1402 may be organized into one or more arrays or clusters that may be linear or nonlinear in nature and may take the form of complex shapes and curves/splines across planar traction pattern 1400. In some embodiments, projections 1402 may be of uniform shape and/or size or may vary along planar traction pattern 1402. Recesses 1404 may also be included. Planar traction pattern 1400 may include one or more extended surfaces 1410. Extended surface 1410 may be raised or lowered relative to the general plane of the traction pattern. As shown in FIG. 14, for example, extended surface 1410 may be a connected surface, and in cooperation with one or more of the features (e.g., projections 1402 and recesses 1404) may denote a pattern (in FIG. 14, a generally wave-like pattern).

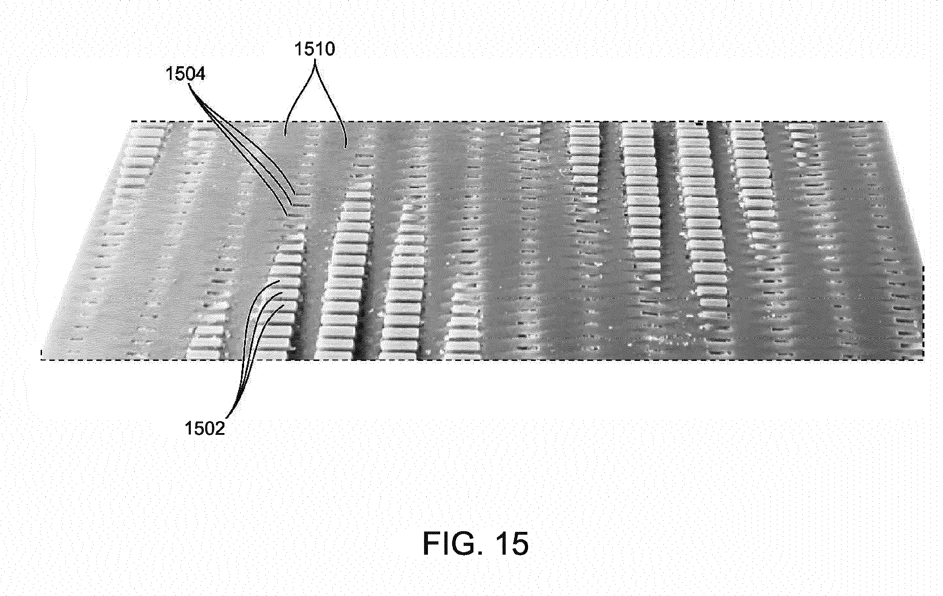

FIG. 15 shows an exemplary planar traction pattern in an intermediate state of manufacture. As shown, the beginning features of projections 1502, recesses 1504, and planar surfaces 1510 are shown taking shape, along with the generally wave-like pattern.

In comparing FIGS. 12-15, especially, themes and variations in the traction profiles can be found, particularly with relationship to the various elements, and general wave-like pattern shown. Indeed, the variations in their respective projections, recesses, fins, planar surfaces, including their shapes, dimensions, number, contours, geometries, and arrangements may be accounted for with a custom computational traction profile. That is, computational traction profile may adjust these features and produce the planar traction patterns 1200, 1300, 1400, and 1500, based on various parametric or algorithmic inputs (e.g., as determined by sensor module 102, data analysis system 200, data translation system 300, or manufacturing system 400.

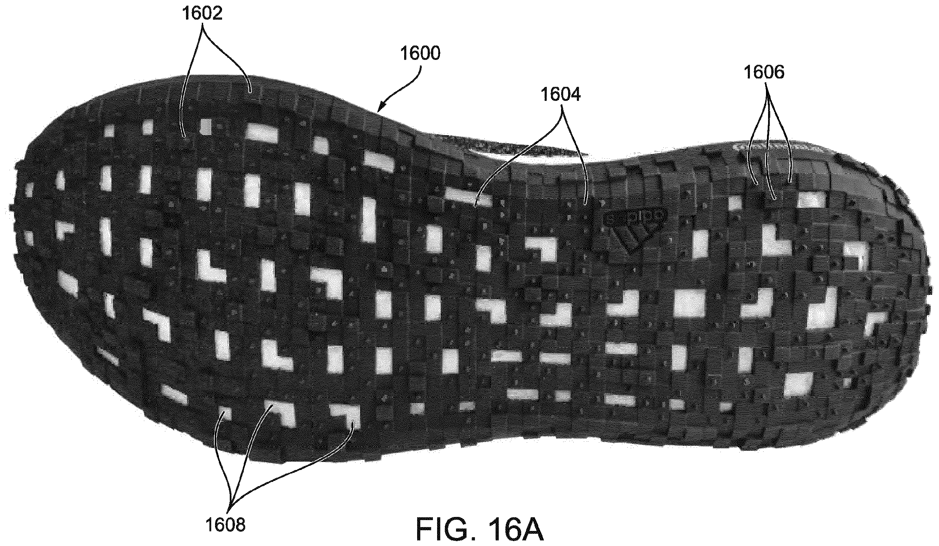

FIGS. 16A through 28 show exemplary whole or partial outsoles 1600, 1700, 1800, 1900, 2000, 2100, 2200, 2300, 2400, 2500, 2600, 2700, and 2800 having outsoles formed using computational traction profiles to alter a visual outsole pattern.

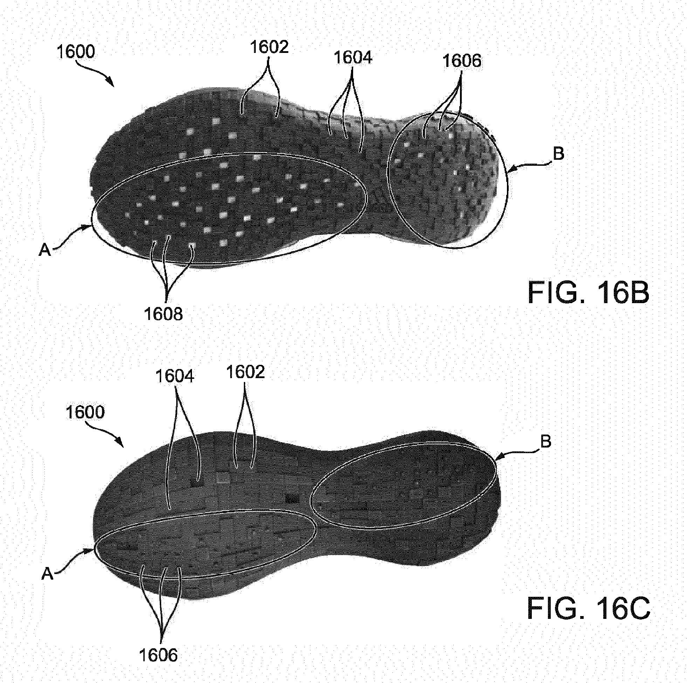

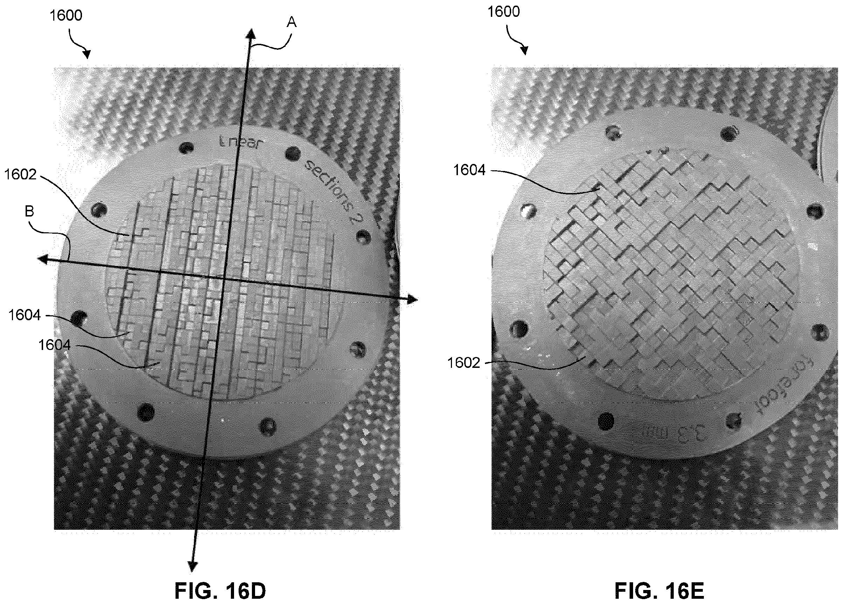

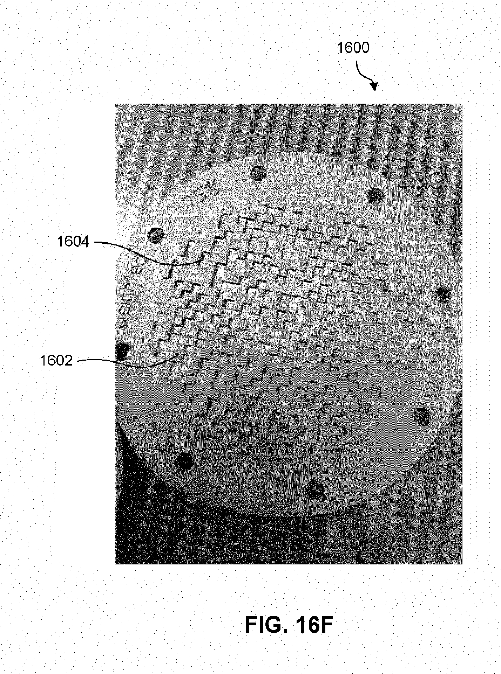

Turning to FIGS. 16A, 16B, 16C, 16D, 16E, and 16F, traction patterns applied to an outsole 1600, having varying patterns according to an embodiment are shown. As shown, outsole 1600 may include projections 1602, and recesses 1604. In some embodiments, outsole 1600 may include apertures 1608, which may expose a midsole or insole, for example in a finished shoe. In some embodiments, outsole 1600 may further include protrusions 1606 that may extend from a surface of projection 1602. In some embodiments, the projections 1602 and protrusions 1606 may form a piexelated pattern, having smaller "pixels" in one or more regions and/or larger pixels in one or more regions of the outsole.

As with traction profiles shown in FIGS. 12-15, the size, shape, placement, etc. of the various features of the traction pattern on outsole 1600 may be varied according to the computational traction profile. For example, where a higher level of traction may be desirable according to the computational traction profile, projection 1602 and/or protrusion 1606 may increase in height, density per area, concentration, or placement. Where less traction is needed, additional recesses 1604 and/or apertures 1608 may be provided. The embodiment shown in FIGS. 16A, 16B, and 16C, are each based on a base traction pattern. With different computational traction profile applied to the pattern, for example through data translation system 300, a visual outsole pattern may be generated, and then manufactured as described herein. This results in three different, customized outsoles, for example for different individuals or different activities. In some embodiments, outsole 1600 may be characterized through multiple zones, for example zone A and zone B, shown in FIGS. 16B and 16C.

FIG. 16D shows a linear configuration for outsole 1600 in which the directionality of the projections 1602 and recesses 1604 affects the traction. Specifically, in this embodiment, projections 1602 and recesses 1604 are grouped in linear rows in the direction of arrow A. Certain linear rows include relatively more projections 1602 than recesses 1604, and those may be alternated with the row made up relatively more recesses 1604 than projections 1602. This configuration provides a stronger coefficient of traction in a linear direction (i.e., the direction of arrow A) compared to a lateral direction (i.e., the direction of arrow B). FIG. 16E shows a configuration for outsole 1600 that improves all-around traction in multiple directions. In this embodiments, there are relatively more projections 1602 than recesses 1604 overall, which improves the traction. In some embodiments, the height, density per area, concentration, and/or placement of projections 1602 are varied and results in improved traction. For example, projections 1602 may be concentrated in specific areas to provide improved traction in those areas. FIG. 16F shows a configuration for outsole 1600 that provides relatively less traction in multiple directions compared to the configurations in FIGS. 16D and 16E. In this embodiment, projections 1602 are not aligned linearly as in FIG. 16D or concentrated as in FIG. 16E. Rather, there is a more even mix of projections 1602 and recesses 1604 across outsole 1600. The differing traction patterns such as those shown in FIGS. 16A-16F have functional advantages, for example providing high traction specific for all running conditions.

Elements of outsole 1600 may include any suitable cross-sectional shape, such as but not limited to a triangular shape, a square shape, a hexagonal shape, a circular shape, an oval shape, or any other polygonal shape, or one or more combinations of the above shapes. These features may be configured as textured/haptic elements, traction elements, and/or wear resistant elements, for example. In some embodiments, projections 1602 and/or protrusions 1608 may be configured as cleats. Each of the outsoles described herein include one or more portions defining ground contacting surface, and one or more portions defining a perimeter side portion of sole. The protrusions of projection elements may be configured as traction elements and may be provided in a heel portion, a midfoot portion, and/or a forefoot portion of outsole, for example. In some embodiments, traction elements may be disposed continuously from a heel side to a forefoot side of outsole. In some embodiments, traction elements may include cleats.

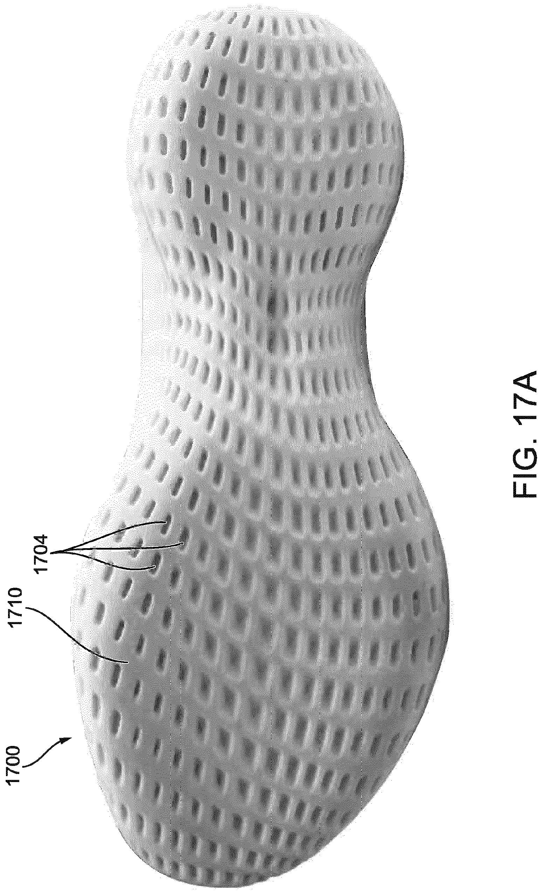

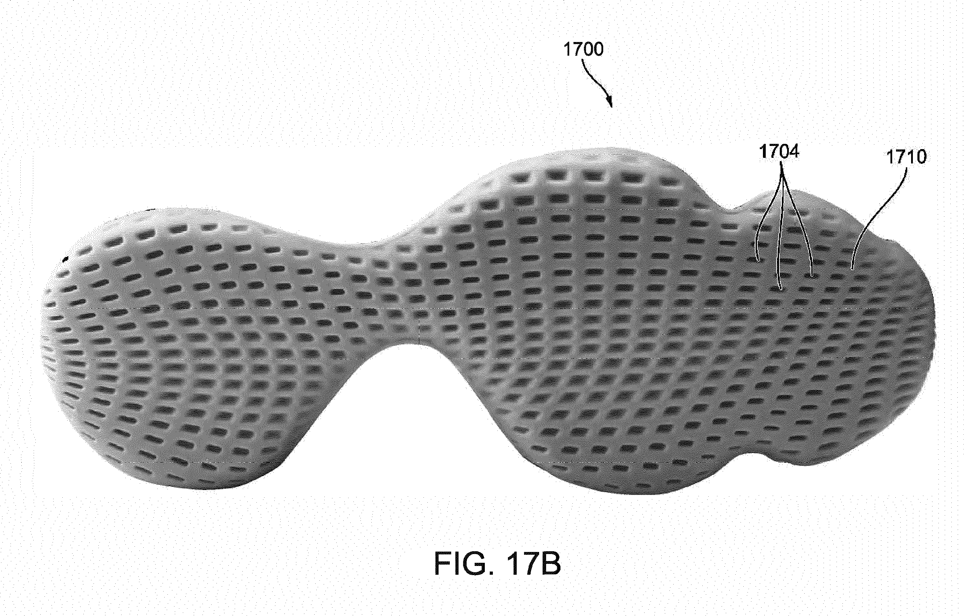

Turning to FIGS. 17A and 17B, traction patterns applied to an outsole 1700, having varying patterns according to an embodiment are shown. As shown, outsole 1700 may include recesses 1704 and extended surfaces 1710. As with the other embodiments, recesses 1704 and extend surfaces 1710 may be positioned in a varying pattern along the surface of outsole 1700, based on computational traction profile. In some embodiments, an overall perimeter shape of outsole 1700 may be varied, for example as a result of the computational traction pattern. Comparing FIG. 17A and 17B, FIG. 17B includes additional curves and contours along its perimeter. Additionally, recesses 1704 may include varying dimensions, concentrations, placements, etc. Traction patterns such as those shown in FIGS. 17A and 17B have functional advantages, for example providing high abrasion resistance. The abrasion resistance can be customized based on high-use areas, for example by including high abrasion resistance in areas where toe dragging typically occurs and more quickly wears down the outsole.

As above, some or all of the features in each traction profile shown in the figures, including those shown in whole or partial outsole embodiments, may be applied in each of the other figures without limitation. For example, features, elements, and properties discussed with reference to outsole 1600 may be used in outsole 1800, for example.

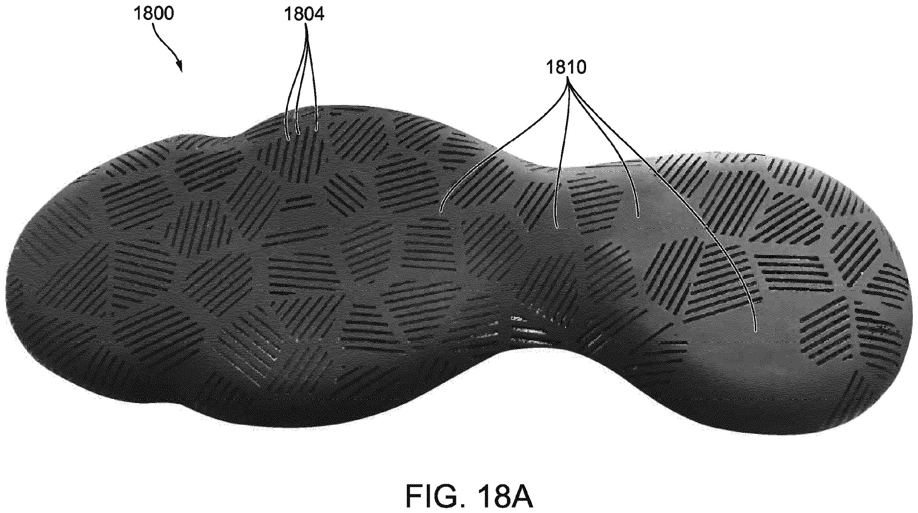

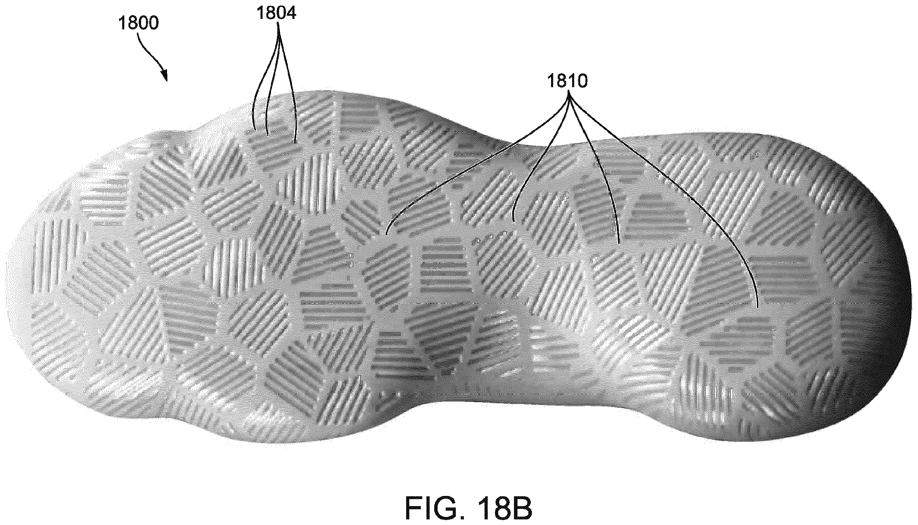

Turning to FIGS. 18A and 18B, traction patterns applied to an outsole 1800, having varying patterns according to an embodiment are shown. As shown, outsole 1800 may include recesses 1804 and extended surfaces 1810. As with the other embodiments, recesses 1804 and extended surfaces 1810 may be positioned in a varying pattern along the surface of outsole 1800, based on computational traction profile. In some embodiments, an overall perimeter shape of outsole 1800 may be varied, for example as a result of the computational traction pattern. Recesses 1804 may be formed, for example by laser cutting. The depth of recesses 1804 may be configured as slits, and may be varied, either from recess to recess, or based on the particular cluster of recesses, as shown in the figure. In some embodiments, a larger cluster of slits may be formed in one region of the outsole compared to another. Slits may also be interconnected to form a continuous pattern, can be intermittent, or both. Comparing FIG. 18A and 18B, FIG. 18A includes additional extended surfaces 1810, where recesses 1804 are not filled in the outline of the shapes. Additionally, recesses 1804 may include varying dimensions, concentrations, placements, etc.

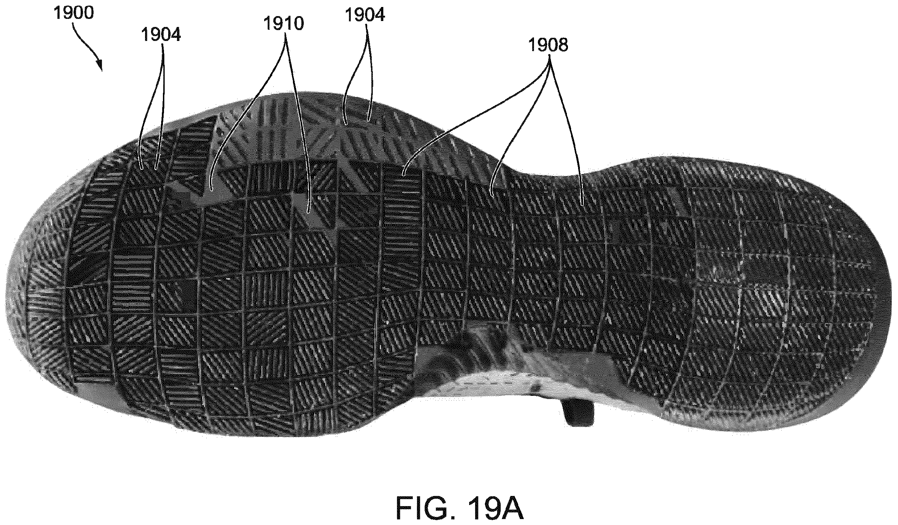

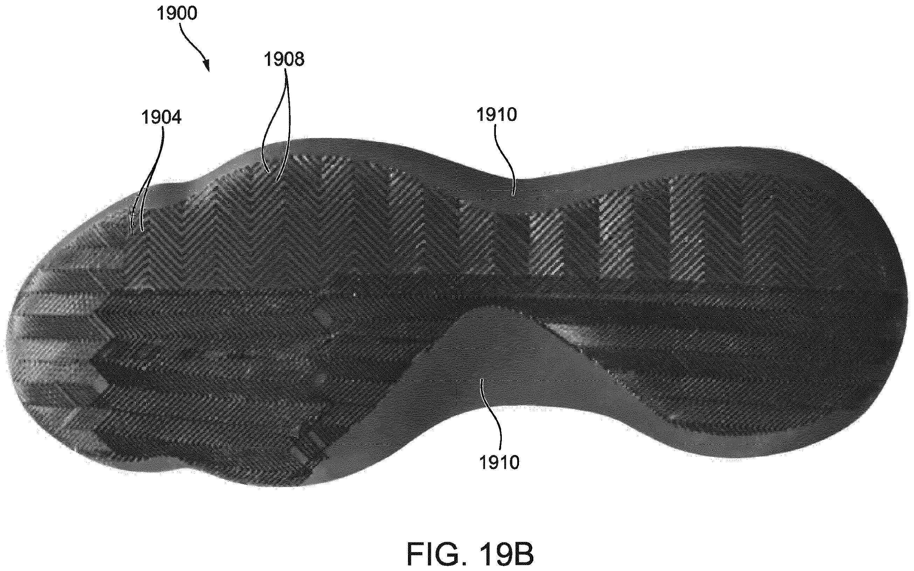

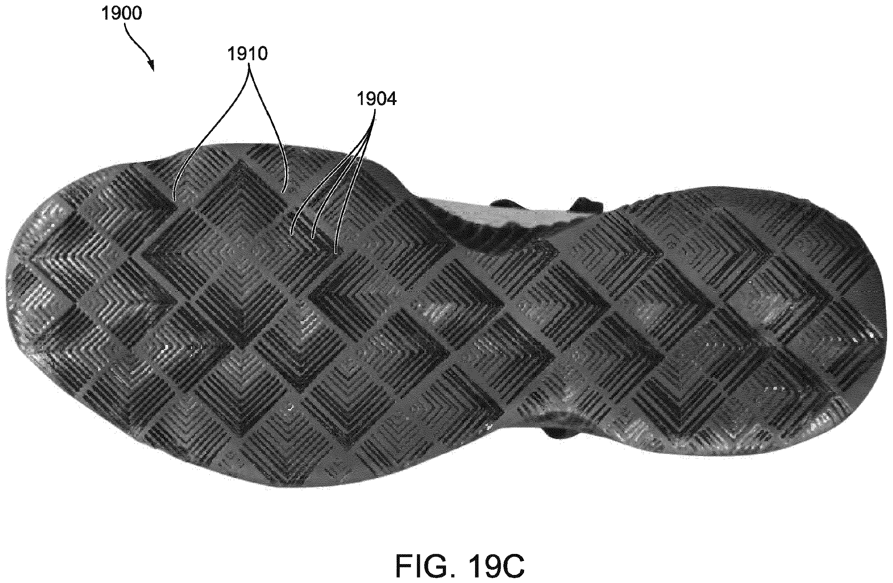

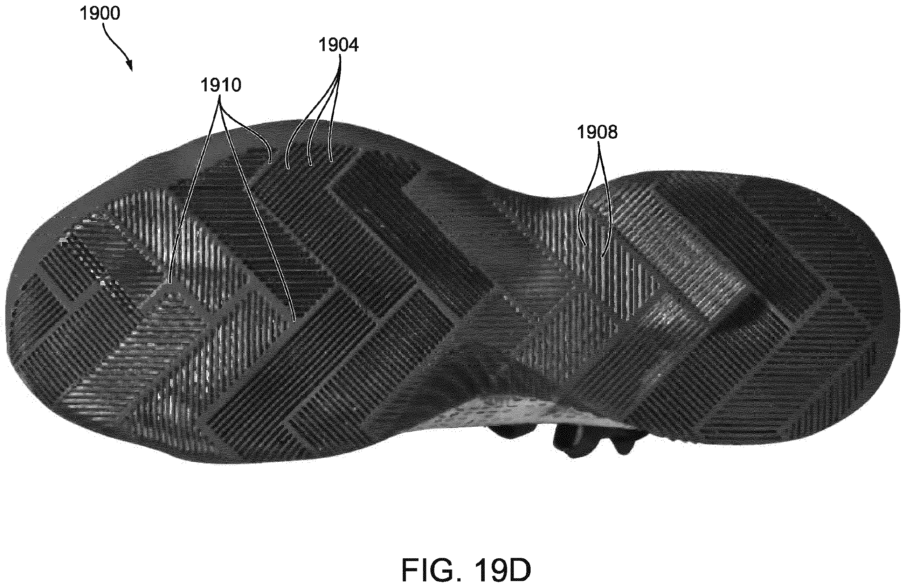

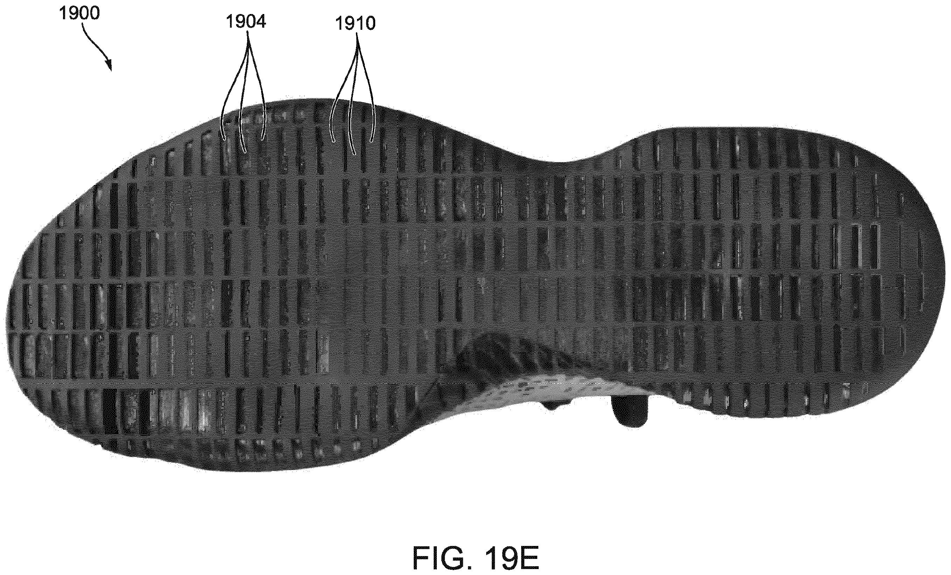

Turning to FIGS. 19A, 19B, 19C, 19D, and 19E traction patterns applied to an outsole 1900, having varying patterns according to an embodiment are shown. As shown, outsole 1900 may include recesses 1904 and extended surfaces 1910. Each of FIGS. 19A, 19B, 19C, 19D, and 19E may include one or more zones with related elements. As with the other embodiments, recesses 1904 and extended surfaces 1910 may be positioned in a varying pattern along the surface of outsole 1900, based on computational traction profile. In some embodiments, an overall perimeter shape of outsole 1900 may be varied, for example as a result of the computational traction pattern. Recesses 1904 may be formed, for example by laser cutting. The depth of recesses 1904 may be configured as slips, and may be varied, either from recess to recess, or based on the particular cluster of recesses, as shown in the figure.

Comparing FIG. 19A and 19B, FIG. 19A includes additional extended surfaces 1910, where recesses are have not filled in the outline of the shapes. Additionally, recesses 1904 may include varying dimensions, concentrations, placements, etc. as shown in FIG. 19A, outsole 1900 may include fin elements 1908. In some embodiments, fin elements 1908 may define shapes, such as grids that separate clusters of recesses 1904, or extended surfaces 1910. As shown, clusters of recesses 1904 may be configured having varying directions, in response to a computational traction profile. As shown, an outer perimeter and heel of outsole 1900 in FIG. 19A shows areas with less recesses, indicating that the computational traction profile may not increase traction in those areas.

As shown in FIG. 19B, recesses 1904 may alternate in a v-shaped or Chevron pattern, for example. FIG. 19C shows a variation in the traction pattern, showing nested right angles in an angle-iron shaped pattern. In some embodiments, the height or profile of the recesses 1904 may be varied, for example to provide additional traction at specific locations according to the computational traction pattern. FIG. 19D shows a variation where fin elements 1908 are nested within extended surfaces in alternating directions, providing a variation on a chevron and alternating fin/recess pattern. FIG. 19E shows a simplified recess pattern, showing varying lengths, widths, and depths of recesses 1904 and extended surfaces 1910.

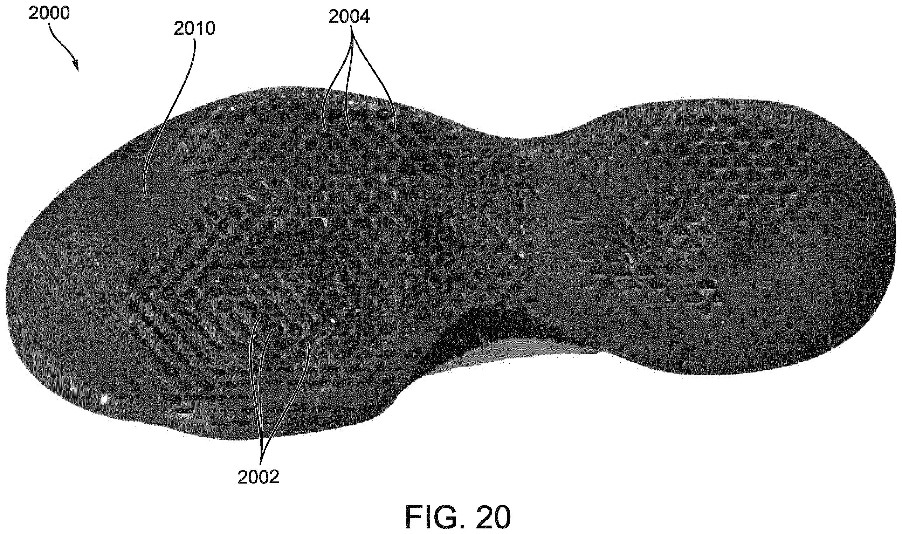

Turning to FIG. 20, traction patterns applied to an outsole 2000, having varying patterns according to an embodiment are shown. As shown, outsole 2000 may include recesses 2004, projections 2002, and extended surfaces 2010. As with the other embodiments, recesses 2004 and extended surfaces 2010 may be positioned in a varying pattern along the surface of outsole 2000, based on computational traction profile. In some embodiments, an overall perimeter shape of outsole 2000 may be varied, for example as a result of the computational traction pattern.

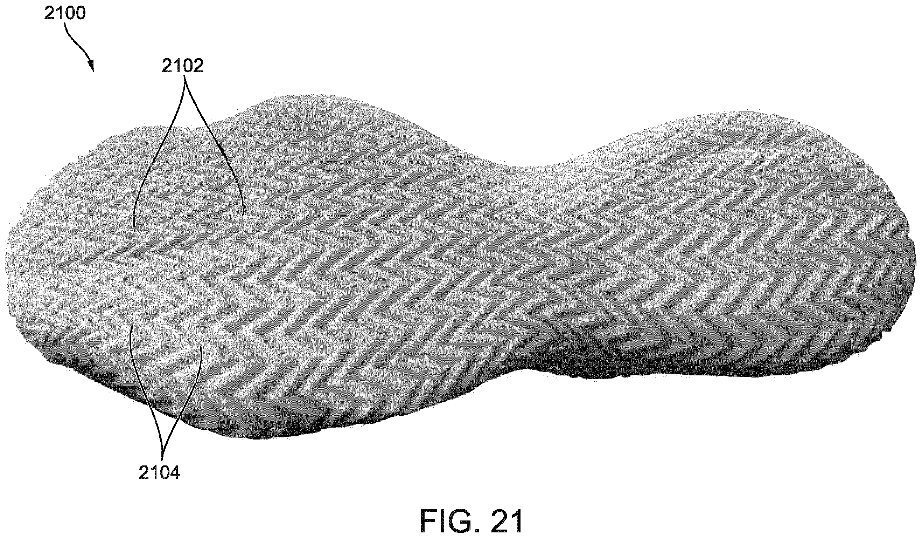

Turning to FIG. 21, traction patterns applied to an outsole 2100, having varying patterns according to an embodiment are shown. As shown, outsole 2100 may include recesses 2104, projections 2102, and extended surfaces 2110. As with the other embodiments, recesses 2104 and projections 2102 may be positioned in a varying pattern along the surface of outsole 2100, based on computational traction profile. In some embodiments, an overall perimeter shape of outsole 2100 may be varied, for example as a result of the computational traction pattern. FIG. 21 shows a repeating zig-zag pattern that may be modified by computational traction profile.

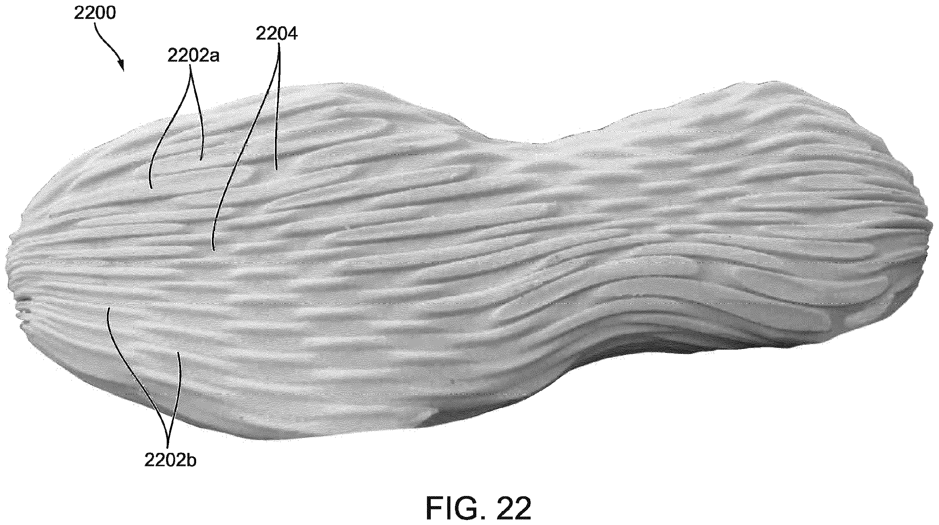

Turning to FIG. 22, traction patterns applied to an outsole 2200, having varying patterns according to an embodiment are shown. As shown, outsole 2200 may include recesses 2204, first projections 2202a and second projections 2202b. As with the other embodiments, recesses 2204 and the projections may be positioned in a varying pattern along the surface of outsole 2200, based on computational traction profile. In some embodiments, an overall perimeter shape of outsole 2200 may be varied, for example as a result of the computational traction pattern. FIG. 22 shows a generally wavy pattern having relatively thicker first projections 2202a and relatively thinner projections 2202b, having varying lenths and profiles that may be modified by computational traction profile.

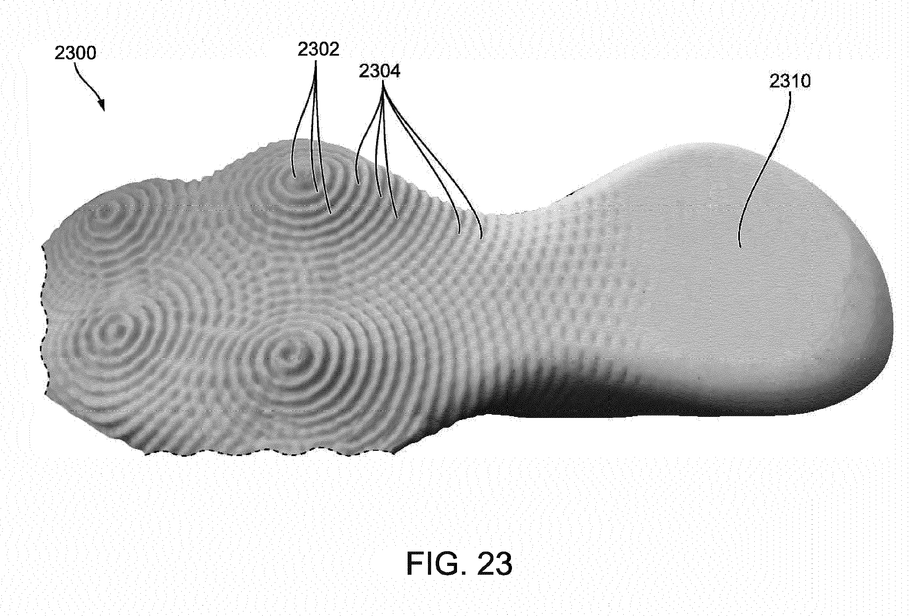

Turning to FIG. 23, traction patterns applied to an outsole 2300, having varying patterns according to an embodiment are shown. As shown, outsole 2300 may include recesses 2304, projections 2302, and extended surfaces 2310. These features may be positioned in a varying pattern along the surface of outsole 2300, based on computational traction profile. In some embodiments, an overall perimeter shape of outsole 2300 may be varied, for example as a result of the computational traction pattern. FIG. 23 shows a generally circular pattern with extending projections and recesses rising and falling and intersecting in outwardly radiating circular shapes. As shown, extended surface 2310 extends over much of the heel of the outsole 2300.

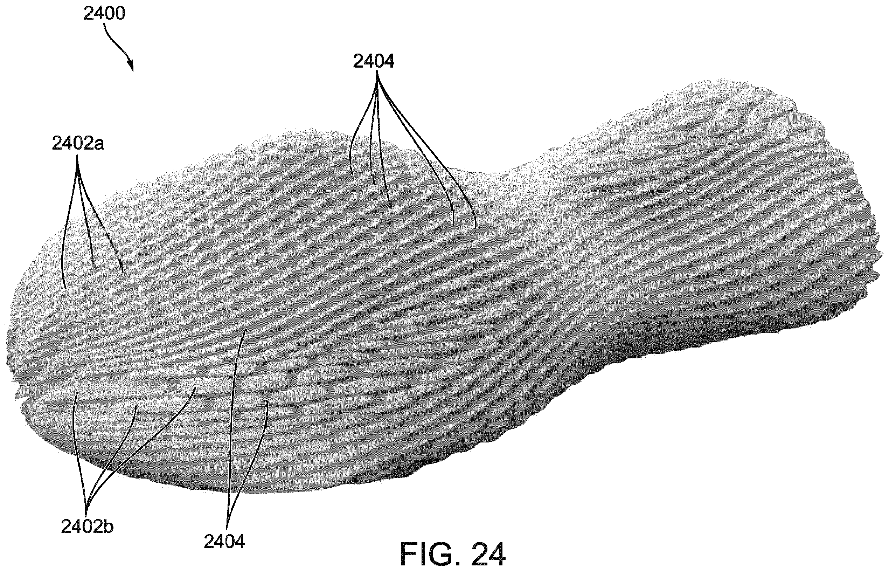

Turning to FIG. 24, traction patterns applied to an outsole 2400, having varying patterns according to an embodiment are shown. As shown, outsole 2400 may include recesses 2404, first projections 2402a and second projections 2402b. As with the other embodiments, recesses 2404 and the projections may be positioned in a varying pattern along the surface of outsole 2400, based on computational traction profile. In some embodiments, an overall perimeter shape of outsole 2400 may be varied, for example as a result of the computational traction pattern. FIG. 24 shows a generally wavy pattern having relatively thicker first projections 2402a and relatively thinner projections 2402b, having varying lengths and profiles that may be modified by the computational traction profile. As shown, first projections 2402a may be formed as a raised fin shape, e.g., as a tooth shaped projection.

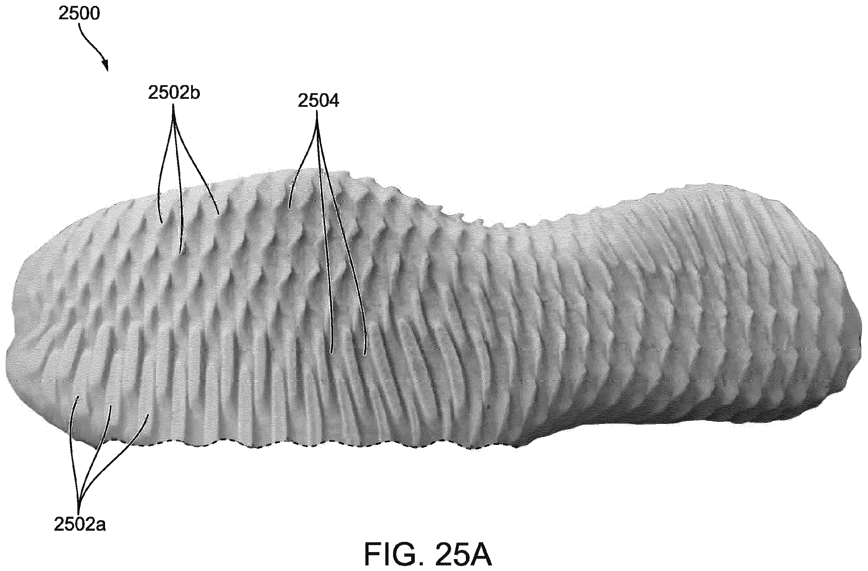

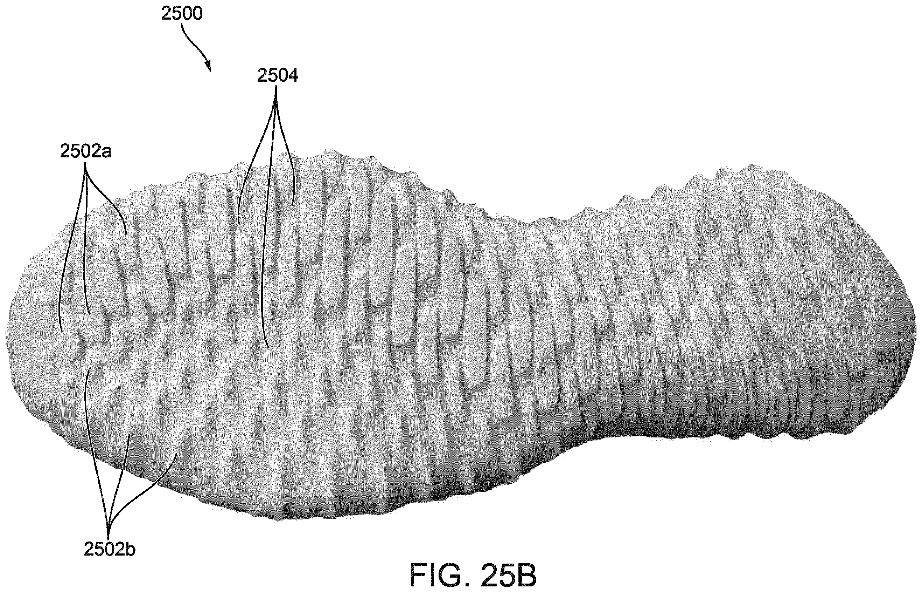

Turning to FIGS. 25A and 25B, traction patterns applied to an outsole 2500, having varying patterns according to an embodiment are shown. As shown, outsole 2500 may include recesses 2504, first projections 2502a and second projections 2502b. As with the other embodiments, recesses 2504 and the projections may be positioned in a varying pattern along the surface of outsole 2500, based on computational traction profile. In some embodiments, an overall perimeter shape of outsole 2500 may be varied as a result of the computational traction pattern. FIG. 25 shows a generally wavy pattern having relatively thicker first projections 2502a and relatively thinner projections 2502b, having varying lengths and profiles that may be modified by computational traction profile. As shown, first projections 2502a may be formed as a raised fin shape, e.g., as a tooth shaped projection. Comparing FIGS. 25A and 25B, first and second projections are provided on opposing sides, e.g., as a mirror image, which may be modified by the computational traction profile.

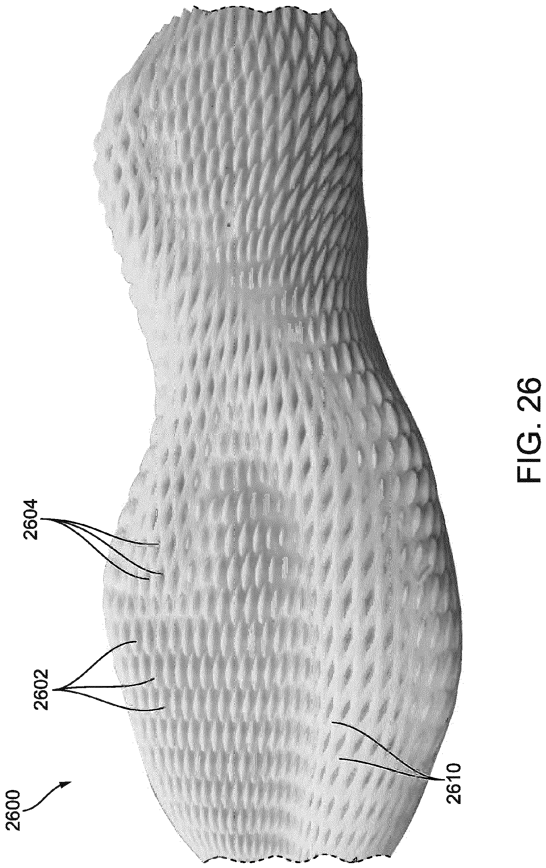

Turning to FIG. 26, traction patterns applied to an outsole 2600, having varying patterns according to an embodiment are shown. As shown, outsole 2600 may include recesses 2604, projections 2602, and extended surfaces 2610. These features may be positioned in a varying pattern along the surface of outsole 2600, based on computational traction profile. In some embodiments, an overall perimeter shape of outsole 2600 may be varied as a result of the computational traction pattern. FIG. 26 shows a pattern having varying surfaces where recesses form a general pattern on inner heel and medial surfaces, contoured towards the outer edge of the blade of the foot. Projections are disposed in other areas, having varying shapes and directions. As with the other embodiments' features, the pattern, direction, number, etc. may all vary in response to the computational traction profile.

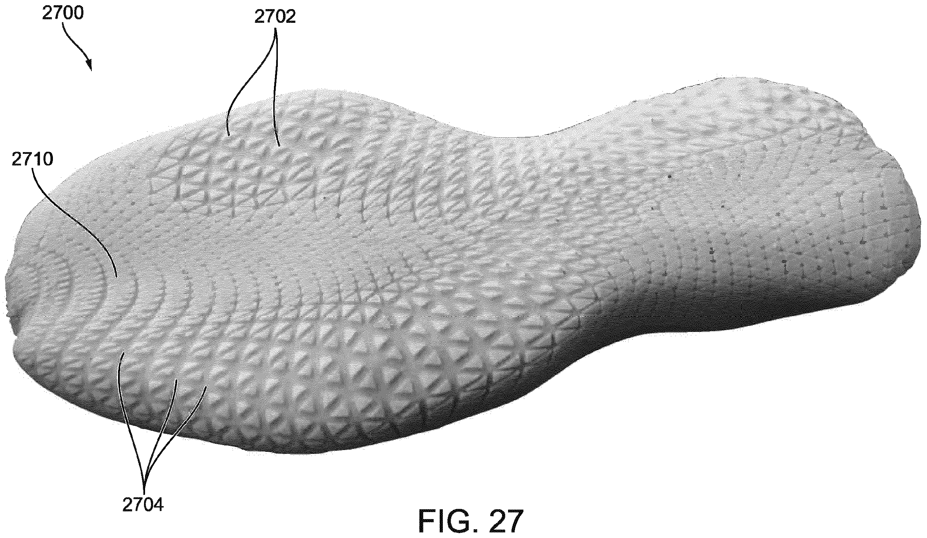

Turning to FIG. 27, traction patterns applied to an outsole 2700, having varying patterns according to an embodiment are shown. As shown, outsole 2700 may include recesses 2704, projections 2702, and extended surfaces 2710. These features may be positioned in a varying pattern along the surface of outsole 2700, based on computational traction profile. In some embodiments, an overall perimeter shape of outsole 2700 may be varied, for example as a result of the computational traction pattern. FIG. 27 shows a pattern having varying surfaces projections are generally triangular in cross section, and recesses are disposed in varying depths and distances from one another. The projection pattern is similar to that in FIG. 26, showing a contoured pattern of projections at outer edges and inner heel locations on outsole 2700. As shown, extended surface 2710 extends over a portion of the heel and towards the toe of the outsole 2700.

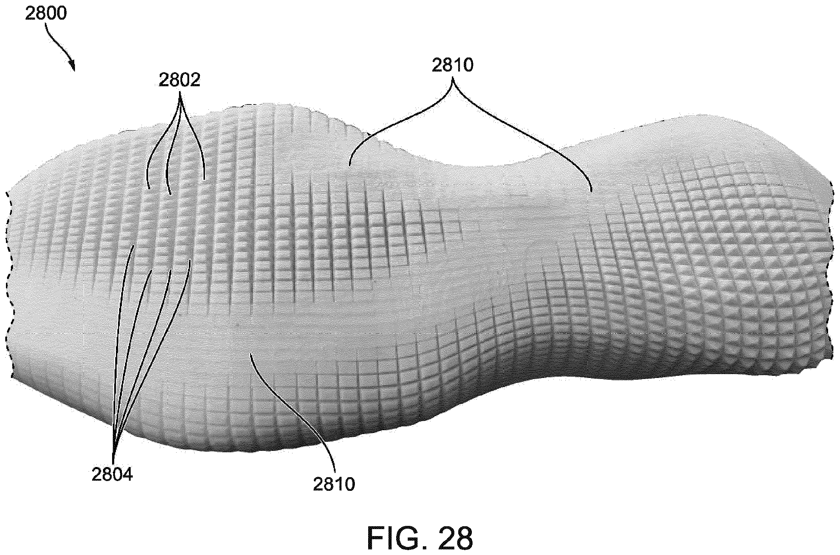

Turning to FIG. 28, traction patterns applied to an outsole 2800, having varying patterns according to an embodiment are shown. As shown, outsole 2800 may include recesses 2804, projections 2802, and extended surfaces 2810. These features may be positioned in a varying pattern along the surface of outsole 2800, based on computational traction profile. In some embodiments, an overall perimeter shape of outsole 2800 may be varied, for example as a result of the computational traction pattern. FIG. 28 shows a pattern having varying surfaces projections are generally prismatic, e.g., a pyramidal or cubic prism shape, and recesses are disposed in varying depths and distances from one another. The projection pattern is similar to that in FIG. 26, showing a contoured pattern of projections at outer edges and heel locations on outsole 2800. As shown, extended surface 2810 extends over a portion of the heel and towards the toe of the outsole 2800, and the extended surface may be formed by portions where the line is blurred between projections 2802 and recesses 2804.

One or more aspects of the methods of manufacturing an outsole for an article of footwear discussed herein, or any part(s) or function(s) thereof, may be implemented using hardware, software modules, firmware, tangible computer readable media having instructions stored thereon, or a combination thereof and may be implemented in one or more computer systems or other processing systems.

FIG. 29 illustrates an exemplary computer system 2900 in which embodiments, or portions thereof, may be implemented as computer-readable code. For example, aspects of the methods discussed herein that may be implemented in one or more computer systems include, but are not limited to, collecting a data via sensor module 102, analyzing data via data system 200, translating data via data translation system 300, and making a custom product via manufacturing system 400, may be implemented in computer system 2900 using hardware, software, firmware, tangible computer readable media having instructions stored thereon, or a combination thereof and may be implemented in one or more computer systems or other processing systems. In all embodiments, a computational traction profile may be customized and generated, leading to the creation of a customized outsole.

If programmable logic is used, such logic may execute on a commercially available processing platform or a special purpose device. One of ordinary skill in the art may appreciate that embodiments of the disclosed subject matter can be practiced with various computer system configurations, including multi-core multiprocessor systems, minicomputers, and mainframe computers, computer linked or clustered with distributed functions, as well as pervasive or miniature computers that may be embedded into virtually any device.

For instance, at least one processor device and a memory may be used to implement the above described embodiments. A processor device may be a single processor, a plurality of processors, or combinations thereof. Processor devices may have one or more processor "cores."

Various embodiments of the inventions may be implemented in terms of this example computer system 2900. After reading this description, it will become apparent to a person skilled in the relevant art how to implement one or more of the inventions using other computer systems and/or computer architectures. Although operations may be described as a sequential process, some of the operations may in fact be performed in parallel, concurrently, and/or in a distributed environment, and with program code stored locally or remotely for access by single or multiprocessor machines.

Processor device 2904 may be a special purpose or a general-purpose processor device. As will be appreciated by persons skilled in the relevant art, processor device 2904 may also be a single processor in a multi-core/multiprocessor system, such system operating alone, or in a cluster of computing devices operating in a cluster or server farm. Processor device 2904 is connected to a communication infrastructure 2906, for example, a bus, message queue, network, or multi-core message-passing scheme.

Computer system 2900 also includes a main memory 2908, for example, random access memory (RAM), and may also include a secondary memory 2910. Secondary memory 2910 may include, for example, a hard disk drive 2912, or removable storage drive 2914. Removable storage drive 2914 may include a floppy disk drive, a magnetic tape drive, an optical disk drive, a flash memory, a Universal Serial Bus (USB) drive, or the like. The removable storage drive 2914 reads from and/or writes to a removable storage unit 2918 in a well-known manner. Removable storage unit 2918 may include a floppy disk, magnetic tape, optical disk, etc. which is read by and written to by removable storage drive 2914. As will be appreciated by persons skilled in the relevant art, removable storage unit 2918 includes a computer usable storage medium having stored therein computer software and/or data.

Computer system 2900 (optionally) includes a display interface 2912 (which can include input and output devices such as keyboards, mice, etc.) that forwards graphics, text, and other data from communication infrastructure 2906 (or from a frame buffer not shown) for display on display unit 2930.

In alternative implementations, secondary memory 2910 may include other similar means for allowing computer programs or other instructions to be loaded into computer system 2900. Such means may include, for example, a removable storage unit 2922 and an interface 2920. Examples of such means may include a program cartridge and cartridge interface (such as that found in video game devices), a removable memory chip (such as an EPROM, or PROM) and associated socket, and other removable storage units 2922 and interfaces 2920 which allow software and data to be transferred from the removable storage unit 2922 to computer system 2900.

Computer system 2900 may also include a communication interface 2924. Communication interface 2924 allows software and data to be transferred between computer system 2900 and external devices. Communication interface 2924 may include a modem, a network interface (such as an Ethernet card), a communication port, a PCMCIA slot and card, or the like. Software and data transferred via communication interface 2924 may be in the form of signals, which may be electronic, electromagnetic, optical, or other signals capable of being received by communication interface 2924. These signals may be provided to communication interface 2924 via a communication path 2926. Communication path 2926 carries signals and may be implemented using wire or cable, fiber optics, a phone line, a cellular phone link, an RF link or other communication channels.

In this document, the terms "computer program medium" and "computer usable medium" are used to generally refer to media such as removable storage unit 2918, removable storage unit 2922, and a hard disk installed in hard disk drive 2912. Computer program medium and computer usable medium may also refer to memories, such as main memory 2908 and secondary memory 2910, which may be memory semiconductors (e.g. DRAMs, etc.).

Computer programs (also called computer control logic) are stored in main memory 2908 and/or secondary memory 2910. Computer programs may also be received via communication interface 2924. Such computer programs, when executed, enable computer system 2900 to implement the embodiments as discussed herein. In particular, the computer programs, when executed, enable processor device 2904 to implement the processes of the embodiments discussed here. Accordingly, such computer programs represent controllers of the computer system 2900. Where the embodiments are implemented using software, the software may be stored in a computer program product and loaded into computer system 2900 using removable storage drive 2914, interface 2920, and hard disk drive 2912, or communication interface 2924.

Embodiments of the inventions also may be directed to computer program products comprising software stored on any computer useable medium. Such software, when executed in one or more data processing device, causes a data processing device(s) to operate as described herein. Embodiments of the inventions may employ any computer useable or readable medium. Examples of computer useable mediums include, but are not limited to, primary storage devices (e.g., any type of random access memory), secondary storage devices (e.g., hard drives, floppy disks, CD ROMS, ZIP disks, tapes, magnetic storage devices, and optical storage devices, MEMS, nanotechnological storage device, etc.).

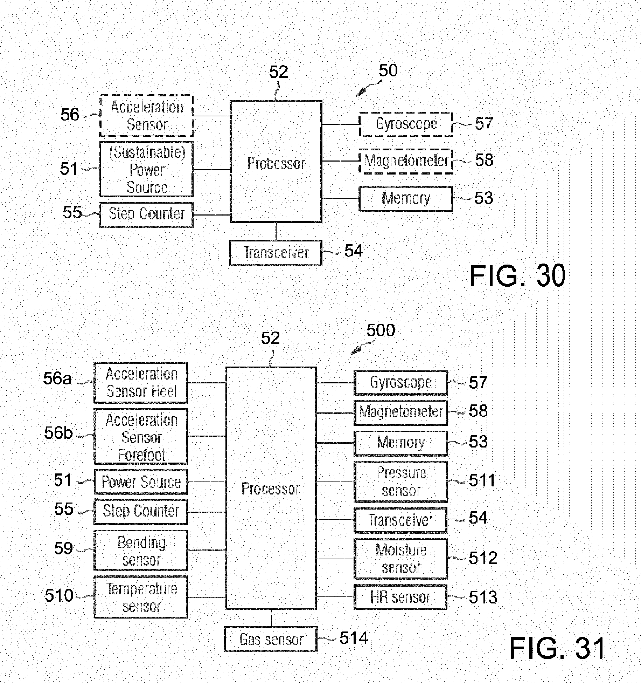

Additionally, in addition to FTIR systems, sensor modules 102 may be embodied as sensors 50 and 500, shown in FIGS. 30 and 31, respectively.

FIG. 30 is a schematic illustration of a sensor device 50 (that may be removable or not removable) integrated or attached into a sports shoe for obtaining sensor data to be used in the context of the present invention. To supply electric power to the electronic components, a power source 51 is integrated into the shoe. This power source could be a battery or rechargeable battery which could be removable. An example of a sustainable power source is a piezo element which generates electric power from pressure variations during running or walking with the sports shoe, a technique which is denoted as energy harvesting.

The sensor device 50 in the example of FIG. 30 may comprise a processor 52 for preprocessing or processing sensor data received from one or more sensors, like described below. For example, the processor 52 could preprocess or evaluate the sensor data and/or perform a gait cycle analysis as described before. The processor 52 is connected to a memory 53 for storing computer instructions and/or data. For example, the received sensor data could be stored in the memory 53 either in raw format or after preprocessing by the processor. Also, results of the gait cycle analysis could be stored in the memory 53.

To transmit the sensor data, results of a gait cycle analysis, etc. from the shoe to another device for creating a digital model of a customized shoe or processing performance data out of the sensor data, the sensor device 50 comprises a transceiver 54. The transceiver 54 could be a Bluetooth, BTLE, Wi-Fi, NFC, cellular network transceiver or the like. It would also be possible to exchange the transceiver with a transmitter to transmit the data like described above. Also, it is possible to transmit the data via a wired connection in which case the transceiver 54 would be a driver for a USB or serial connection for example.

In a minimal setup, the shoe may include a step counter 55 which is able to count the steps taken by the user while he/she is running or walking. Using data from the step counter 55 it is at least possible to determine whether the user is an intensive runner, e.g., how often he/she runs and how long such a run lasts on average. Furthermore, it is possible to extract the pace, i.e. steps per minute. This information may be used in the context of the present invention to build a digital model of a customized sports shoe and to manufacture such a customized shoe based on the digital model, e.g., by altering a custom computational traction profile. If, for example, the user turns out to be an intensive runner with a high pace, the customized shoe could be provided with more or less cushioning characteristics to avoid injuries. Additionally, more traction elements could be added, moved, reshaped, resized, etc., in order to provide particular traction patterns for specific movements. The step counter may be based on an accelerometer and/or at least one piezo element.

In a more advanced setup, the shoe is equipped with an acceleration sensor 56 and a gyroscope 57 as indicated by the dashed boxes. Data from these sensors allow a more advanced analysis of the person's gait cycle. Such a setup uses the lowest power for a gait cycle analysis. The power for such a setup could be provided by energy harvesting techniques as described above. In an alternative setup the shoe comprises an acceleration sensor 56 and a magnetometer 58 to allow for a gait cycle analysis. It is also possible to have all three sensors, i.e. acceleration sensor 56, gyroscope 57 and magnetometer 58 integrated into a shoe which would deliver best results for a gait cycle analysis. In general, it is possible to use only one of the sensors shown FIG. 30, two of those sensors or all three. Additional examples of energy harvesting techniques that may be used are described in commonly owned U.S. Pat. App. No. 15/416, 593, filed January 26, 2017 and published as U.S. 2017/0208890.

FIG. 31 shows an example of a more complex sensor device 500 (also either removable or not removable) integrated or attached to a sport shoe according to the present invention. This example includes a power source 51, processor 52, memory 53, transceiver 54 and step counter 55, gyroscope 57 and magnetometer 58 similar function as described to the setup shown Fig. 5. Additionally, the example of FIG. 30 may include separate acceleration sensors for the heel of the foot and for the forefoot, 56a and 56b, respectively. Thus, acceleration may be measured more accurately and differences between forefoot and heel acceleration may deliver more detailed information for building a digital model of a customized shoe according to the invention. In general, according to the invention any number (including just one) and combination of the described sensors may be used. Thus, not all sensors shown in FIG. 31 may be present and/or used.

Additional sensors such as bending sensor 59 may be included. Such a sensor is able to measure the bending of the shoe, e.g. of its sole. A temperature sensor 510 may measure the outside air temperature and/or the temperature of the person's body or the temperature in the shoe when wearing the shoe.

Pressure sensor 511 is a pressure sensor matrix as described herein. Using such a pressure sensor matrix, it is possible to obtain a series of "pressure maps" (similar to so-called "heat maps") that encode the dynamics of how the foot behaves while moving, e.g., the distribution of pressure over time. This information may enable real-time interactions with the user like for example advises to correct movement while running to prevent injuries.

Moisture sensor 512 is able to measure the moisture inside the shoe and/or outside. Moisture data from inside the shoe may provide information about the person's perspiration. If perspiration is high, the customized shoe may be provided with moisture wicking materials and/or good air ventilation. Moisture data from outside the shoe may indicate whether the person typically runs in bad weather conditions such as rain. If this is the case, the customized shoe may be provided with water repellent coatings.

Heart rate (HR) sensor 513 is able to measure the person's heart rate which may allow to infer the person's fitness level.

Gas sensor 514 may give an indication about the sweat consistence. This may give an indication whether the person is sick (e.g. having athlete's foot (Fuβpilz in German)). In a shirt, the gas sensor 514 could give an indication about the smell level, which then could an indication for the health status.

The physiological characteristics collected by the system may include, but are not limited to, gait characteristics, such as foot strike type (e.g. heel, midfoot, forefoot, etc.), rate of pronation or supination, and degree of pronation and supination. In some embodiments, the system may receive personal information about the individual before or after receiving physiological characteristics data about the individual. Personal information may include information such as their name, prior injury information, height, weight, gender, shoe size, an athletic goal, intended athletic environment or terrain, intended athletic activity duration, intended athletic activity frequency, intended athletic activity distance, quantitative or qualitative preferences about athletic equipment or footwear (such as level of cushion, preference of weight, materials and the like), and current athletic footwear.

In some embodiments, the system may receive biometric data via a local wired or wireless connection. In some embodiments the system may monitor and receive data from an individual in real time during an athletic activity, such as jogging.

Sensor module 102 may include one or more temperature sensors, a heart rate monitoring device, a pedometer, and/or an accelerometer-based monitoring device. Accelerometer, pressure sensor, force sensor, optical stress/strain sensor, temperature sensor, chemical sensor, global positioning system, piezoelectric sensor, rotary position sensor, magnetometer, gyroscopic sensor, heart-rate sensor, goniometer, electrocardiogram (ECG) sensor, electrodermograph (EDG) sensor, electroencephalogram (EEG) sensor, electromyography (EMG) sensor, feedback thermometer sensor, photoplethysmograph sensor, plethysmograph sensor, moisture sensor(s), skin conductance sensor, galvanic skin response (GSR) sensor, electrodermal response (EDR) sensor, psychogalvanic reflex (PGR) sensor, skin conductance response (SCR) sensor, skin conductance level (SCL), sensor or the like.

Sensors of sensor module 102 may be capable of measuring a variety of athletic performance parameters. The term "performance parameters" may include physical parameters and/or physiological parameters associated with the individual's athletic activity. Physical parameters measured may include, but are not limited to, time, distance, speed, pace, pedal count, wheel rotation count, rotation generally, stride count, stride length, airtime, stride rate, altitude, temperature, strain, impact force, jump force, force generally, and jump height. Physiological parameters measured may include, but are not limited to, heart rate, respiration rate, blood oxygen level, blood lactate level, blood flow, hydration level, calories burned, or body temperature.

Data collected by sensor module 102 may classify individuals based on their running style, utilizing data analysis such as an anterior-posterior plot angle vs. time; medial-lateral plot angle vs. time; and the like. Calculations of these characteristic many be used to group individuals into different categories (groups), such as a heel striker, a mid foot striker, a forefoot striker, a pronator, supinator, a neutral individual, or some combination of characteristics. In some embodiments, gait analysis may utilize personal information of individual 102, such a gender, shoe size, height, weight, running habits, and prior injuries.

In some embodiments, a regression analysis can be used to determine gait characteristics such as foot strike type, rate of pronation, degree of pronation, and the like based on acceleration data obtained from sensor module 102. In some embodiments, the regression analysis can be used to determine gait characteristics such as foot strike type, rate of pronation, degree of pronation, and the like based on other data such as magnetometer data, angular momentum sensor data, or multiple types of data. In some embodiments, the analysis can include other userinput information such as prior injury information, an athletic goal, intended athletic environment or terrain, intended athletic duration, and current athletic footwear.

Athletic goals may be, for example, training for a race, to stay healthy, to lose weight, and training for sports. Other examples of athletic goals may include training for a race, or other sporting event, improving individual fitness, simply enjoy running, or the like. Frequency intervals may include for example about 1 to 2 times per week, about 3 to 4 times per week, about 5 to 7 times per week, or the individual doesn't know. Length intervals may include for example about less than about 5 miles (SI Unit: 1609 m = 1 mile) per week, about 5 to 10 miles per week, about 10 to 20 miles per week, greater than about 20 miles per week, or the individual doesn't know. Examples of intended athletic terrain environments may include roads, track, treadmill, trail, gym, or particular athletic fields designed for a specific sport. Examples of athletic equipment preferences may include for example more cushioning, less weight, better fit, strength, durability, intended athletic activity range, balance, weight balance, more color choices, and the like.

In any of the various embodiments discussed herein, the method may further comprise receiving second light intensity data obtained by the sensor module on which an individual performs a second activity wearing the outsole; identifying whether a performance goal has been met or not met; and in response to identifying that a performance goal has not been met, updating the computational traction profile and visual outsole pattern; and producing a second outsole based on the visual outsole pattern.

In any of the various embodiments discussed herein, the method may further comprise receiving personal information about the individual prior to receiving the data about the individual.

In any of the various embodiments discussed herein, the producing the outsole may comprise laser-cutting, 3D-printing, or 3D-molding.

In any of the various embodiments discussed herein, the method may be performed in a retail store.

In any of the various embodiments discussed herein, the method may further comprise receiving data obtained by a second sensor module used by the individual in an athletic activity and updating the computational traction profile in response to the data obtained by the second sensor module.

In any of the various embodiments discussed herein, the second sensor module may comprise an instrumented insole.

In any of the various embodiments discussed herein, the article of footwear comprises an outsole, wherein one of the material, thickness, stiffness, cushioning properties, abrasion resistance, or traction pattern of the outsole is determined in response to the received sensor data.

In any of the various embodiments discussed herein, the article of footwear may further comprise a midsole, wherein one of the material, thickness, stiffness, insulation, or cushioning properties of the midsole is determined in response to the received sensor data.

In any of the various embodiments discussed herein, the article of footwear comprises an outsole comprising a traction element, wherein the position of the traction element determined in response to the received sensor data.

In any of the various embodiments discussed herein, the sensor data comprises light intensity data over a period of time, and is captured during a particular movement by the individual.

In any of the various embodiments discussed herein, the article of footwear may further comprise an outsole comprising projections, wherein the position of the projections along a surface of the outsole is varied in response to the computational traction profile.

In any of the various embodiments discussed herein, the article of footwear may further comprise an outsole comprising projections, wherein a height of the projections is varied in response to the computational traction profile.

In any of the various embodiments discussed herein, the article of footwear may further comprise an outsole comprising projections, wherein a crosssectional shape of the projections is varied in response to the computational traction profile.

In any of the various embodiments discussed herein, the second sensor data is obtained from a sensor module different from a sensor module that obtains the first sensor data.

In any of the various embodiments discussed herein, the second sensor data is obtained during an athletic activity.

In any of the various embodiments discussed herein, the first sensor module comprises a frustrated total internal reflection ("FTIR") system, and the second sensor module may comprise an instrumented insole.

Various aspects of the present invention, or any parts or functions thereof, may be implemented using hardware, software, firmware, tangible nontransitory computer readable or computer usable storage media having instructions stored thereon, or a combination thereof and may be implemented in one or more computer systems or other processing systems.

Program products, methods, and systems of the present invention can include any software application executed by one or more electronic devices. An electronic device can be any type of computing device having one or more processors. For example, the electronic device can be a workstation, mobile device (e.g., a mobile phone, personal digital assistant, tablet computer, or laptop), computer, server, compute cluster, server farm, game console, set-top box, kiosk, embedded system, a gym machine, a retail system or retail enhancement system or other device having at least one processor and memory. Embodiments of the present invention may be software executed by a processor, firmware, hardware or any combination thereof in a computing device.

In this document, terms such as "computer program medium" and "computer-usable medium" may be used to generally refer to media such as a removable storage unit or a hard disk installed in hard disk drive. Computer program medium and computer-usable medium may also refer to memories, such as a main memory or a secondary memory, which can be memory semiconductors (e.g., DRAMs, etc.). These computer program products provide software to computer systems of the present invention.

Computer programs (also called computer control logic) may be stored on main memory and/or secondary memory. Computer programs may also be received via a communications interface. Such computer programs, when executed, may enable computer systems of the present invention to implement embodiments described herein. Where embodiments are implemented using software, the software can be stored on a computer program product and loaded into a computer system using, for example, a removable storage drive, an interface, a hard drive, and/or communications interface.

Based on the description herein, a person skilled in the relevant art will recognize that the computer programs, when executed, can enable one or more processors to implement processes described above, such as the steps in the methods illustrated by the figures. In some embodiments, the one or more processors can be part of a computing device incorporated in a clustered computing environment or server farm. Further, in some embodiments, the computing process performed by the clustered computing environment may be carried out across multiple processors located at the same or different locations.

Software of the present invention may be stored on any computer-usable medium. Such software, when executed in one or more data processing device, causes the data processing device to operate as described herein. Embodiments of the invention employ any computer-usable or -readable medium, known now or in the future. Examples of computer-usable mediums include, but are not limited to, primary storage devices (e.g., any type of random access or read only memory), secondary storage devices (e.g., hard drives, floppy disks, CD ROMS, ZIP disks, tapes, magnetic storage devices, optical storage devices, MEMS, nanotechnological storage devices, memory cards or other removable storage devices, etc.), and communication mediums (e.g., wired and wireless communications networks, local area networks, wide area networks, intranets, etc.).

While various embodiments of the present invention have been described above, they have been presented by way of example only, and not limitation. It should be apparent that adaptations and modifications are intended to be within the meaning and range of equivalents of the disclosed embodiments, based on the teaching and guidance presented herein. It therefore will be apparent to one skilled in the art that various changes in form and detail can be made to the embodiments disclosed herein without departing from the scope of the claimed invention. The elements of the embodiments presented above are not necessarily mutually exclusive, but may be interchanged to meet various needs as would be appreciated by one of skill in the art.

It is to be understood that the phraseology or terminology used herein is for the purpose of description and not of limitation. The breadth and scope of the present invention should not be limited by any of the above-described exemplary embodiments but should be defined only in accordance with the following claims.

It is to be appreciated that the Detailed Description section, and not the Summary and Abstract sections, is intended to be used to interpret the claims. The Summary and Abstract sections may set forth one or more but not all exemplary embodiments of the present invention as contemplated by the inventor(s).

## Claims

1. A method (1100) of manufacturing an outsole, comprising:
receiving light intensity data (1102) obtained by at least one sensor module on which an individual performs an activity,
wherein a portion of the received sensor data is obtained by a frustrated total internal reflection, FTIR, system having a surface on which an individual may perform a movement;
correlating (1104) the received light intensity data with a traction characteristic;
building a computational traction profile in response to the correlation;
creating (1106), in response to the computational traction profile, a visual outsole pattern; and
producing (1108) the outsole based on the visual outsole pattern.

2. The method (1100) of claim 1, further comprising:
receiving second light intensity data obtained by the sensor module on which an individual performs a second activity wearing the outsole;
identifying whether a performance goal has been met or not met; and
in response to identifying that a performance goal has not been met, updating the computational traction profile and visual outsole pattern; and
producing a second outsole based on the visual outsole pattern.

3. The method (1100) of claim 1, further comprising receiving personal information about the individual prior to receiving the data about the individual.

4. The method (1100) of claim 1, wherein producing the outsole comprises laser-cutting, 3D-printing, or 3D-molding.

5. The method (1100) of claim 1, further comprising:
receiving data obtained by a second sensor module used by the individual in an athletic activity; and
updating the computational traction profile in response to the data obtained by the second sensor module.

6. The method (1100) of claim 5, wherein the second sensor module comprises an instrumented insole.

7. The method of claim 1, wherein the method is performed in a retail store.

8. A method of generating a visual outsole pattern, comprising:
producing a visual outsole pattern in a 3D-environment;
receiving sensor data correlated with traction of an outsole having a physical traction pattern,
wherein a portion of the received sensor data is obtained by a frustrated total internal reflection, FTIR, system having a surface on which an individual may perform a movement;
building a computational traction profile based on the received sensor data; and
updating the visual outsole pattern based on the computational traction profile.

9. The method of claim 8, further comprising:
manufacturing a physical outsole as modeled by the visual outsole pattern;
receiving second sensor data correlated with traction of the physical outsole;
updating the computational traction profile based on the received second sensor data; and
updating the visual outsole pattern based on the computational traction profile.

10. The method of claim 9, wherein the second sensor data is obtained from a sensor module different from a sensor module that obtains the first sensor data.

11. The method of claim 10, wherein the second sensor data is obtained during an athletic activity.

12. The method of claim 10, wherein the sensor module that obtains the first sensor data comprises a frustrated total internal reflection, FTIR, system, and wherein the sensor module that obtains the second sensor data comprises an instrumented insole.

## Patentansprüche

1. Verfahren (1100) zum Herstellen einer Außensohle, umfassend:
Empfangen von Lichtintensitätsdaten (1102), die von mindestens einem Sensormodul erhalten werden, auf dem eine Person eine Aktivität ausführt,
wobei ein Teil der empfangenen Sensordaten durch ein frustriertes internes Totalreflexionssystem, FTIR-System, mit einer Oberfläche erhalten wird, auf der eine Person eine Bewegung ausführen kann;
Korrelieren (1104) der empfangenen Lichtintensitätsdaten mit einer Traktionscharakteristik;
Erstellen eines rechnerischen Traktionsprofils als Reaktion auf die Korrelation;
Erstellen (1106) eines visuellen Außensohlenmusters als Reaktion auf das rechnerische Traktionsprofil; und
Herstellen (1108) der Außensohle basierend auf dem visuellen Außensohlenmuster.

2. Verfahren (1100) nach Anspruch 1, ferner umfassend:
Empfangen von zweiten Lichtintensitätsdaten, die von dem Sensormodul erhalten werden, auf dem eine Person eine zweite Aktivität ausführt, die die Außen sohle trägt;
Identifizieren, ob ein Leistungsziel erreicht wurde oder nicht; und
Aktualisieren des rechnerischen Traktionsprofils und des visuellen Außensohlenmusters als Reaktion auf das Identifizieren, dass ein Leistungsziel nicht erreicht wurde; und
Herstellen einer zweiten Außensohle basierend auf dem visuellen Außensohlen muster.

3. Verfahren (1100) nach Anspruch 1, ferner umfassend das Empfangen von persönlichen Informationen über die Person vor dem Empfangen der Daten über die Person.

4. Verfahren (1100) nach Anspruch 1, wobei das Herstellen der Außensohle Laserschneiden, 3D-Drucken oder 3D-Formen umfasst.

5. Verfahren (1100) nach Anspruch 1, ferner umfassend:
Empfangen von Daten, die von einem zweiten Sensormodul erhalten werden,
das von der Person bei einer sportlichen Aktivität verwendet wird; und
Aktualisieren des rechnerischen Traktionsprofils als Reaktion auf die Daten, die von dem zweiten Sensormodul erhalten werden.

6. Verfahren (1100) nach Anspruch 5, wobei das zweite Sensormodul eine instrumentierte Einlegesohle umfasst.

7. Verfahren nach Anspruch 1, wobei das Verfahren in einem Einzelhandelsgeschäft ausgeführt wird.

8. Verfahren zum Erzeugen eines visuellen Außensohlenmusters, umfassend:
Herstellen eines visuellen Außensohlenmusters in einer 3D-Umgebung;
Empfangen von Sensordaten, die mit der Traktion einer Außensohle korreliert sind, die ein physisches Traktionsmuster aufweist,
wobei ein Teil der empfangenen Sensordaten durch ein frustriertes internes Totalreflexionssystem, FTIR-System, mit einer Oberfläche erhalten wird, auf der eine Person eine Bewegung ausführen kann;
Erstellen eines rechnerischen Traktionsprofils basierend auf den empfangenen Sensordaten; und
Aktualisieren des visuellen Außensohlenmusters basierend auf dem rechnerischen Traktionsprofil.

9. Verfahren nach Anspruch 8, ferner umfassend:
Herstellen einer physischen Außensohle, die durch das visuelle Außensohlenmuster modelliert wird;
Empfangen von zweiten Sensordaten, die mit der Traktion der physischen Außensohle korreliert sind;
Aktualisieren des rechnerischen Traktionsprofils basierend auf den empfangenen zweiten Sensordaten; und
Aktualisieren des visuellen Außensohlenmusters basierend auf dem rechnerischen Traktionsprofil.

10. Verfahren nach Anspruch 9, wobei die zweiten Sensordaten von einem Sensormodul erhalten werden, das sich von einem Sensormodul unterscheidet, das die ersten Sensordaten erhält.

11. Verfahren nach Anspruch 10, wobei die zweiten Sensordaten während einer sportlichen Aktivität erhalten werden.

12. Verfahren nach Anspruch 10, wobei das Sensormodul, das die ersten Sensordaten erhält, ein frustriertes internes Totalreflexionssystem, FTIR-System, umfasst und wobei das Sensormodul, das die zweiten Sensordaten erhält, eine instrumentierte Einlegesohle umfasst.

## Revendications

1. Un procédé (1100) de fabrication d'une semelle extérieure, comprenant :
la réception de données d'intensité lumineuse (1102) obtenues par au moins un module détecteur sur lequel un individu se livre à une activité,
dans lequel une partie des données de détecteur reçues est obtenue par un système à réflexion interne totale contrariée, FTIR, possédant une surface sur laquelle un individu peut exécuter un mouvement ;
la corrélation (1104) des données d'intensité lumineuse reçues avec une caractéristique de traction ;
la construction d'un profil de traction calculé, en réponse à la corrélation ;
la création (1106), en réponse au profil de traction calculé, d'un modèle visuel de semelle extérieure ; et
la production (1108) de la semelle extérieure, sur la base du modèle visuel de semelle extérieure.

2. Le procédé (1100) de la revendication 1, comprenant en outre :
la réception de secondes données d'intensité lumineuse obtenues par le module détecteur sur lequel un individu se livre à une seconde activité en portant la semelle extérieure ;
l'identification si un but de performance a été atteint ou non atteint ; et
en réponse à une identification qu'un but de performance n'a pas été atteint, la mise à jour du profil de traction calculé et du modèle visuel de semelle extérieure ; et
la production d'une seconde semelle extérieure, sur la base du modèle visuel de semelle extérieure.

3. Le procédé (1100) de la revendication 1, comprenant en outre la réception d'informations personnelles relatives à l'individu avant la réception des données relatives à l'individu.

4. Le procédé (1100) de la revendication 1, dans lequel la production de la semelle extérieure comprend une découpe laser, une impression 3D, ou un moulage 3D.

5. Le procédé (1100) de la revendication 1, comprenant en outre :
la réception de données obtenues par un second module détecteur utilisé par l'individu dans une activité athlétique ; et
la mise à jour du profil de traction calculé, en réponse aux données obtenues par le second module détecteur.

6. Le procédé (1100) de la revendication 5, dans lequel le second module détecteur comprend une semelle intérieure instrumentée.

7. Le procédé de la revendication 1, dans lequel le procédé est mis en oeuvre dans une boutique d'un revendeur.

8. Un procédé de génération d'un modèle visuel de semelle extérieure, comprenant :
la production d'un modèle visuel de semelle extérieure dans un environnement 3D ;
la réception de données de détecteur corrélées avec la traction d'une semelle extérieure présentant un modèle physique de traction,
dans lequel une partie des données de détecteur reçues est obtenue par un système à réflexion interne totale contrariée, FTIR, possédant une surface sur laquelle un individu peut exécuter un mouvement ;
la construction d'un profil de traction calculé, sur la base des données de détecteur reçues ; et
la mise à jour du modèle visuel de semelle extérieure, sur la base du profil de traction calculé.

9. Le procédé de la revendication 8, comprenant en outre :
la fabrication d'une semelle extérieure physique d'après la maquette du modèle visuel de semelle extérieure ;
la réception de secondes données de détecteur corrélées à la traction de la semelle extérieure physique ;
la mise à jour du profil de traction calculé, sur la base des secondes données de détecteur reçues ; et
la mise à jour du modèle visuel de semelle extérieure, sur la base du profil de traction calculé.

10. Le procédé de la revendication 9, dans lequel les secondes données de détecteur sont obtenues à partir d'un module détecteur différent d'un module détecteur qui obtient les premières données de détecteur.

11. Le procédé de la revendication 10, dans lequel les secondes données de détecteur sont obtenues au cours d'une activité athlétique.

12. Le procédé de la revendication 10, dans lequel le module détecteur qui obtient les premières données de détecteur comprend un système à réflexion interne totale contrariée, FTIR, et dans lequel le module détecteur qui obtient les secondes données de détecteur comprend une semelle intérieure instrumentée.
